# EUROPEAN PATENT APPLICATION

(11) **EP 4 785 780 A1**
(43) Date of publication of application: **05.08.2026**
(21) Application number: 26155976.9
(22) Date of filing: 03.02.2026
(51) Int. Cl.: A01J 5/013, A01J 5/007, A01K 29/00, G01N 33/04, A01K 5/02

(54) **SYSTEM AND METHOD FOR AUTOMATED DETECTION OF METABOLIC DISORDERS IN DAIRY LIVESTOCK BASED ON INLINE MONITORING OF MILK QUALITY**

(30) Priority: 04.02.2025 US 202519044921
(71) Applicant: Technologies Holdings Corporation, Houston, TX 77019 (US)
(72) Inventor: Constantine, Damien, Riverview, E1B 5A8 (CA); Kinney, Angela, Fitchburg, WI 53711 (US); Pretz, Steve, Westwood Hills, KS 66205 (US)
(74) Representative: Sandersons

(57) **Abstract**

A system (100) for automated detection of metabolic condition of dairy livestock is provided, comprising: milking stations (106) associated with milk lines (104); inline sensors (102) each configured to measure milk composition information of milk flowing through an associated milk line (104); and a processor configured to: receive, from each inline sensor (102), milk data indicative of milk composition information of milk flowing through that inline sensor (102) during a milking session; associate the received milk data with a particular animal that produced the milk; store milk composition information for milk from the particular animal during the milking session; determine metabolic condition information for the particular animal using stored milk composition information for at least one milking session; and determine, based on the metabolic condition information, that the particular animal is experiencing a subclinical negative energy balance.

## Description

### BACKGROUND

Currently, it is expensive, time consuming, and impractical to identify every cow that is in the early stages of a metabolic disorder, such as a negative energy balance condition that, if not addressed, may negatively impact milk quality and/or production, and may lead to ketosis, which can negatively impact the health of the cow. For example, in order to detect a negative energy balance in a cow that may negatively impact milk quality and/or production, a farmer can collect milk and/or blood samples from each cow, and send the samples to a lab to be tested for the presence and/or level of analytes indicative of a negative energy imbalance (e.g., non-esterified fatty acids (NEFA), β-hydroxybutyrate (BHB), etc.). Collecting, transport, and testing the samples is time consuming (e.g., requiring at least days to receive results, during which the condition of the cows may have deteriorated further), and costly, and may not detect a condition in a timely manner. Additionally, the cost of monitoring a herd of dairy cows increases with the size of the herd, as more samples, tests, etc., as the number of cows increases. Accordingly, a farmer may not find it economically advantageous to attempt to detect and treat subclinical negative energy balance, which can lead to the health of some cows in a herd deteriorating unnecessarily.

The general industry standard for determining milk quality being produced by cows on a dairy farm is testing based on standards promoted by the Dairy Herd Improvement Association (DHIA testing), and/or similar organizations. For example, DHIA testing generally includes projecting a cow's milk production by taking a sample of milk from each cow one time per month, and analyzing the milk in a laboratory setting to determine various properties of the milk, which results in milk production for most cows being measured only 10 times in a typical 305 lactation period, and a relatively long period of time (e.g., more than a month) during which a cow's condition can deteriorate before a metabolic condition is detected from routine milk analysis.

### SUMMARY

In accordance with some embodiments of the disclosed subject matter, a system for automated detection of metabolic condition of dairy livestock is provided, the system comprising: a plurality of milking stations, each associated with a respective milk line of a plurality of milk lines; a plurality of inline sensors, each configured to measure milk composition information of milk flowing through one of the plurality of milk lines, wherein a first subset of milk lines of the plurality of milk lines are equipped with a respective inline sensor of the plurality of inline sensors, such that milk composition information is generated for milk flowing through the first subset of milk lines during milking; and one or more hardware processors configured to: receive, from each of the plurality of inline sensors, milk data indicative of milk composition information of milk flowing through that inline sensor during a milking session; associate the received milk data with a particular animal that produced the milk; store milk composition information for milk from the particular animal during the milking session; determine metabolic condition information for the particular animal using stored milk composition information for at least one milking session; and determine, based on the metabolic condition information, that the particular animal is experiencing a subclinical negative energy balance.

In some embodiments, the system further comprises: a robotic feeding system configured to dispense feed for consumption by each of a plurality of animals, including the particular animal, wherein the one or more hardware processors are further configured to: in response to determining that the particular animal is experiencing a subclinical negative energy balance and without user intervention, instruct the robotic feeding system to adjust the composition of feed provided to the particular animal to provide additional energy to the particular animal.

In some embodiments, the one or more hardware processors are further configured to: determine a dose of a high energy supplement to provide to the particular animal based on the metabolic condition information; and instruct the robotic feeding system to add an amount of the high energy supplement to feed provided to the particular animal, wherein the amount is based on the dose.

In some embodiments, the high energy supplement comprises propylene glycol.

In some embodiments, the high energy supplement consists essentially of propylene glycol.

In some embodiments, the system further comprises: an activity monitoring sensor configured to measure animal activity information, wherein the one or more hardware processors are further configured to: receive, from the activity monitoring sensor, activity data indicative of activity of the particular animal; store activity information for the particular animal; determine the metabolic condition information for the particular animal using the stored milk composition information for the at least one milking session and stored activity information for over a predetermined period of time that includes the at least one milking session.

In some embodiments, the one or more hardware processors are further configured to: receive, from the activity monitoring sensor, activity data indicative of activity of a second particular animal; store activity information for the second particular animal; determine metabolic condition information for the second particular animal using stored milk composition information for a plurality of milking sessions over a predetermined period of time and stored activity information for the predetermined period of time; determine, based on the metabolic condition information, that the particular animal is not experiencing a subclinical negative energy balance; and instruct a robotic feeding system to provide feed to the second particular animal that does not include an added amount of the high energy supplement.

In some embodiments, the activity monitoring sensor is affixed to the particular animal, and is one of a plurality of activity monitoring sensors, each associated with a different animal of a plurality of animals.

In some embodiments, a second subset of milk lines the plurality of milk lines are not equipped with inline an inline sensor configured to measure milk composition information of milk flowing through one of the second subset of milk lines, such that milk composition information is not generated for milk flowing through the second subset of milk lines during milking, and wherein the number of milk lines in the first subset of milk lines is less than the number of milk lines in the second subset of milk lines.

In some embodiments, each milk line of the plurality of milk lines is a member of the first subset of milk lines of the second subset of milk lines. In some embodiments, each milk line of the plurality of milk lines is a member of the first subset of milk lines or the second subset of milk lines.

In some embodiments, the plurality of milking stations comprise all of the milk stations of a milking operation, such that only a fraction of milking stations of the milking operation are equipped with inline sensors configured to measure milk composition information of milk during milking.

In some embodiments, the first subset of milk lines is about thirty percent of the plurality of milking stations.

In some embodiments, the milking operation comprises a robotic milking system configured to autonomously attach milking devices to animals at the plurality of milking stations for milking.

In some embodiments, the stored milk composition information comprises fat content information for the milking session, protein content information for the milking session, and/or lactose content information for the milking session.

In accordance with some embodiments of the disclosed subject matter, a method for automated detection of metabolic condition of dairy livestock is provided, the method comprising: receiving, from each of a plurality of inline sensors, milk data indicative of milk composition information of milk flowing through that inline sensor during a milking session, wherein each of the plurality of inline sensors is configured to measure milk composition information of milk flowing through one of a plurality of milk lines, which are each associated with a respective milking station of a plurality of milking stations, and wherein a first subset of milk lines of the plurality of milk lines are equipped with a respective inline sensor of the plurality of inline sensors, such that milk composition information is generated for milk flowing through the first subset of milk lines during milking; associating the received milk data with a particular animal that produced the milk; storing milk composition information for milk from the particular animal during the milking session; determining metabolic condition information for the particular animal using stored milk composition information for at least one milking session; and determining, based on the metabolic condition information, that the particular animal is experiencing a subclinical negative energy balance.

### BRIEF DESCRIPTION OF THE DRAWINGS

Various objects, features, and advantages of the disclosed subject matter can be more fully appreciated with reference to the following detailed description of the disclosed subject matter when considered in connection with the following drawings, in which like reference numerals identify like elements.
FIG. 1A shows an example of a system for automated metabolic condition monitoring and treatment of dairy livestock in accordance with some embodiments of the disclosed subject matter.
FIG. 1B shows an example of a portion of a system for monitoring metabolic condition monitoring of dairy livestock using milk quality data from inline sensors in accordance with some embodiments of the disclosed subject matter.
FIG. 2 shows an example of a system for monitoring the metabolic condition of dairy livestock using milk quality data from inline sensors in accordance with some embodiments of the disclosed subject matter.
FIG. 3 shows an example of hardware that can be used to implement a data source, a computing device, and a server in accordance with some embodiments of the disclosure.
FIG. 4 shows an example of a process for automated metabolic condition monitoring and treatment of dairy livestock using inline sensors in accordance with some embodiments of the disclosed subject matter.
FIG. 5 shows an example of a process for automated milk quality monitoring using inline sensors in accordance with some embodiments of the disclosed subject matter.
FIG. 6 shows an example of a process for automated detection of adverse metabolic conditions based on milk quality information in accordance with some embodiments of the disclosed subject matter.
FIG. 7 shows an example flow for automated metabolic condition monitoring and treatment in accordance with some embodiments of the disclosed subject matter.

### DETAILED DESCRIPTION

As described above, attempting to monitor the health of dairy livestock closely enough to detect the early stages of metabolic disorders by manually collecting samples from every animal for analysis is impractical, costly, and time consuming. However, early detection and timely treatment for metabolic disorders can improve the health of lactating dairy animals, leading to improvements in milk quality and/or production, which can have a large impact on the monetary success of a dairy farm, as an animal that declines in health often lead to increased costs (e.g., for veterinary care, more costly treatments at more advanced stages, etc.) and/or decreased revenue (e.g., decreased milk production, low quality milk, etc.). More automated systems for monitoring the health of dairy animals have been developed that utilize an on-site device that uses chemical reagents to test milk in a more practical time period. However, such systems are generally costly to install and maintain, require extensive changes to milk collection systems, and require ongoing purchases of (often costly) reagents to replenish the reagents which are consumed during testing.

In some embodiments, mechanisms described herein can collect milk quality information using inline sensors that measure characteristics of milk during milking as the milk flows from the dairy animal (e.g., the cow) to a milk storage tank, and can use the milk quality information and other health information (e.g., based on activity of the animal throughout the day) to determine whether a particular animal is in a negative energy balance state that may negatively impact the health, milk production, and/or milk quality of the animal, and/or is progressing toward such a state. While installing such an inline sensor on every milk line (or even for every teat of each cow) would be expected to generate the most valuable and timely milk quality data for detection of deteriorating metabolic condition of cows, such inline sensors are costly to purchase and install. In some embodiments, mechanisms described herein can facilitate collection of milk quality information for each cow in dairy operation with a significantly higher sampling frequency than conventional techniques that collect only limited samples. For example, in some embodiments, mechanisms described herein can use a quantity of sensors that is less than the number of milking stalls in a dairy operation, which can be used to statistically quantify milk quality over several milking sessions to a high level of confidence. Unlike conventional monthly sampling, mechanisms described herein can be used continuously, resulting in increased sampling per lactation period. For example, in a typical large herd installation, the number of samples taken can be expected to increase from 10 samples per lactation to about 180 samples per lactation when about 30% of milk stalls are monitored using the inline sensors. Additionally, these samples can be expected to be from various times of day, and mitigates the potential that the owner of the herd may attempt to manipulate data and/or mitigates common sources of human error during sampling (e.g., by mislabeling, mishandling, etc.). Additionally, such inline sensors can use techniques that measure electric and/or optical properties of the milk, rather than using reagents to measure chemical properties of the milk, without ongoing costs associated with replenishing reagents.

As described above, while there are benefits to installing one or more inline sensors for each milk line, there are also costs associated with installing additional sensors, such as a cost to purchase each sensor, a cost to install each sensor (e.g., which may involve electrical work to provide a source of electrical power to each inline sensor), a cost to monitor operation of the inline sensor (e.g., to determine whether data being generated by each inline sensor is relatively accurate), a cost to perform regular schedule maintenance on the sensor (e.g., which may be higher than costs associated with maintaining a milk line that is not provided with an inline sensor(s)), a cost to perform unscheduled maintenance (e.g., if the sensor malfunctions), etc. In such an example, while providing one or more inline sensors for each milk line to facilitate monitoring of milk characteristics for every milking session for every cow is advantageous, purchasing, installing and maintaining that number of sensors may outweigh the benefits for many dairy owner and/or operators. Monitoring milk quality using inline sensors on only a fraction of milking sessions reduces the costs of monitoring, and also does not scale at a one to one cost with increases in the size of the herd. In some embodiments, mechanisms described herein can use milk quality information collected using inline milk quality sensors on a fraction of milk lines to detect cows with subclinical metabolic conditions efficiently, and in real time.

In some embodiments, mechanisms described herein can facilitate more robust monitoring of milk quality and animal health for a herd of dairy livestock, at a reduced cost compared to a system that monitors every milking of every animal. Note that although mechanisms described herein are generally described in connection with milking cows, mechanisms described herein can be used in connection with other types of dairy livestock, such as goats, sheep, etc.

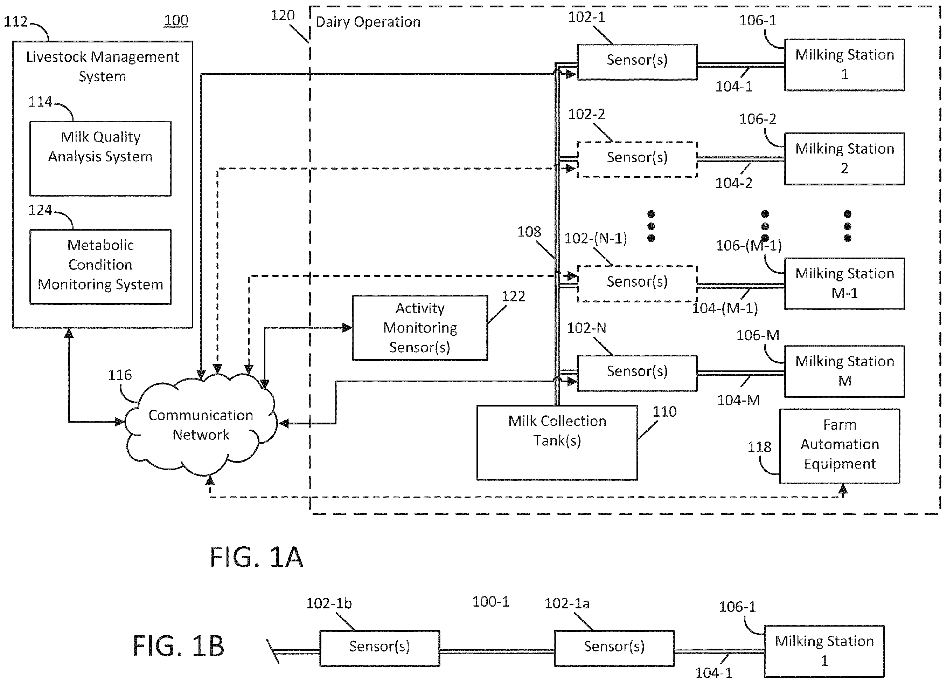

FIG. 1A shows an example of a system 100 for automated metabolic condition monitoring and treatment of dairy livestock in accordance with some embodiments of the disclosed subject matter. As shown in FIG. 1A, system 100 can include a dairy operation 120 which can include any suitable number of milking stations 106 (e.g., M milking stations 106-1 to 106-M), which can each be configured to milk a dairy livestock animal, such as a dairy cow. For example, each milking station 106 can include, or otherwise be associated with, a milking device(s) that can be attached to the teats of a cow to automatically extract milk from the cow. In some embodiments, each milking station can be associated with a milk line 104 (e.g., M milk lines 104-1 to 104-M corresponding to milking stations 106-1 to 106-M) that can be used to direct milk toward one or more milk tanks 110 for collection. In the example of FIG. 1A, multiple milk lines 104 are connected to milk tank 110 via a bulk line 108. However, one or more of milk lines 104-1 to 104-M can be individually connected to milk tank 110, and/or one or groups of milk lines 104-1 to 104-M can each be connected to milk tank 110 via a respective bulk line. Note that although not shown, milk lines 104-1 to 104-M can be connected to multiple milk tanks (e.g., for storing milk with different characteristics) and/or to a waste disposal line (e.g., directed to a waste tank, a sewer line, etc.) via any suitable valves and/or other flow control devices.

In some embodiments, system 100 can include any suitable number of inline sensors 102 (e.g., a number N that is less than the total number of milking stations M), which can each be associated with a particular milking station of milking stations 106-1 to 106-M, and which are configured to analyze fluid flowing through a respective milk line 104-1 to 104-M associated with the associated milking station. For example, inline sensor(s) 102 can be integrated into the associated milk line, and/or can be retrofitted to an existing milk line. In some embodiments, each inline sensor 102 can include a sensor(s) that can be used to determine one or more characteristics of milk flowing through the associated milk line (e.g., inline sensor 102-1 can include a sensor(s) that can be used to analyze milk flowing through milk line 104-1, and can generate data that can be used to determine a characteristic(s) of the milk, as the milk flows through sensor 102-1).

In some embodiments, inline sensor 102 can be configured to generate data that can be used to determine any suitable characteristic(s) of the milk flowing through inline sensor 102. In some embodiments, inline sensor 102 can be configured to generate data that can be used to determine an amount and/or concentration of one or more components of milk, such as one or more main components (e.g., fat. protein, and/or lactose), one or more ancillary and/or trace components (e.g., metabolites and/or other analytes that are generally present in relatively small amounts, etc.), one or more potential contaminants (e.g., blood cells, bacteria, etc.), and/or one or more parameters indicative of an amount of milk flowing through, and/or that has flowed through, the inline sensor and/or milk line. For example, inline sensor 102 can be configured to generate data that can be used to determine milk composition information indicative of the amount (e.g., in grams) and/or concentration (e.g., weight per volume such as milligrams per deciliter (mg/dl), percent by weight, percent by volume, etc.) of main components of milk (e.g., components that are generally expected to make up at least 1% by weight of normal milk), such as fat content, protein content, lactose content, and/or water content of milk flowing through inline sensor 102. As another example, inline sensor 102 can be configured to generate data that can be used to determine milk composition information indicative of an estimated amount and/or concentration (e.g., cells per milliliter (cells/ml), milligrams per deciliter (mg/dl), parts per million, percent by weight, percent by volume, etc.) of ancillary and/or trace components of milk such as concentration of a urea-containing molecule (e.g., milk urea nitrogen (MUN)), somatic cells (e.g., in cells/ml), etc., expected to be present in milk flowing through inline sensor 102. As yet another example, inline sensor 102 can be configured to generate data that can be used to determine milk composition information indicative of an estimated amount and/or concentration (e.g., cells/ml, milligrams per deciliter (mg/dl), parts per million, percent by weight, percent by volume, etc.) of ancillary and/or trace components of milk such as concentration of somatic cells, blood, etc., expected to be present in milk flowing through inline sensor 102.

In some embodiments, inline sensor 102 can include any suitable hardware, firmware, and/or software that can be used to generate data that is suitable for determining a characteristic(s) of the milk flowing through inline sensor 102. For another example, inline sensor 102 can include hardware, firmware, and/or software that can be used to generate optical spectra data indicative of a presence and/or concentration of a particular molecule in the milk flowing through inline sensor 102. In a more particular example, inline sensor 102 can include a wavelength tunable light source (e.g., a swept-source laser, a frequency comb laser, etc.) configured to emit light of a particular wavelength or combination of wavelengths at a particular time toward milk flowing through a portion of inline sensor 102 (e.g., a light source that emits and/or can be controlled to emit a particular wavelength of light at a particular time that is known and/or can be detected, which can be a coherent light source such as a laser) and a detector (e.g., a photodetector or an array of photodetectors, such as a photodetector(s) configured to detect light in a wavelength range of the tunable light source) configured to detect light of a particular wavelength (e.g., based on a time at which the signal was sampled, and/or based on a wavelength of light directed toward the detector which samples the signal, such as via a grating) that has passed through, been reflected by, and/or been emitted by the milk.

As another example, inline sensor 102 can include hardware, firmware, and/or software that can be used to generate optical data indicative of a color(s) of light reflected and/or absorbed by the milk flowing through inline sensor 102. In a more particular example, inline sensor 102 can include a light source configured to emit light toward milk flowing through a portion of inline sensor 102 (e.g., a light source that emits light in multiple wavelengths, which can be a noncoherent light source) and an image sensor(s) (e.g., a color image sensor, such as a 1D or 2D CMOS or CCD sensor array(s) in which each pixel is associated with a particular color filter) configured detect an intensity of light of different colors that has passed through and/or been reflected by the milk passing through inline sensor 102 (e.g., many color image sensors use red, green, and blue filter arrays, but other combinations of filter colors, including some that include white and/or infrared pixels, can be used to characterize the intensity of light of different colors in light detected by an image sensor).

As yet another example, inline sensor 102 can include hardware, firmware, and/or software that can be used to generate electrical data indicative of one or more electric properties of milk flowing through inline sensor 102, such as a conductivity and/or any other suitable electric properties of the milk. In a more particular example, inline sensor 102 can include an electrode(s) configured to emit an electrical signal into milk flowing through a portion of inline sensor 102, and an electrode(s) configured to detect the electrical signal emitted into the milk flowing through the portion of inline sensor 102, and generate data indicative of an electrical property of the milk through which the signal was transmitted.

As still another example, inline sensor 102 can include hardware, firmware, and/or software that can be used to generate data indicative of a temperature of milk flowing through inline sensor 102.

As a further example, inline sensor 102 can include hardware, firmware, and/or software that can be used to generate data indicative of a volume of milk flowing through inline sensor 102.

In some embodiments, sensor 102 can include multiple different types of sensors in a single housing that can be used to generate data indicative of different characteristics of the milk flowing through inline sensor 102. For example, an inline sensor for determining characteristics of milk is described in Buciunas et al. International Patent Application Publication No. WO2024/075079, which can include a color sensor configured to generate color data that can be used to determine a characteristic(s) of milk that impacts color of milk (e.g., milk fat content, the presence of blood in the milk), a laser source and laser detector configured to generate spectral data that can be used to determine a characteristic(s) of milk that impacts how a particular wavelength(s) of light are absorbed and/or reflected by the milk (e.g., the presence and/or concentration of fat molecules, proteins, lactose, urea-containing molecules, etc.), and a contact sensor(s) configured to generate electric data that can that can be used to determine a characteristic(s) of milk (e.g., a concentration of somatic cells) that impacts an electrical property or properties (e.g., conductivity) of the milk, and/or configured to generate temperature data that can be used to more accurately determine a characteristic(s) of milk that is impacted by temperature (e.g., absorption of different wavelengths of light that can vary with concentration of a molecule(s) that absorbs the particular wavelength of light and the temperature of the milk, milk viscosity, temperature compensated conductivity, protein solubility, fat globule behavior, lactose crystallization, enzyme activity such as lactase vs alkaline phosphatase, milk pH, bacterial growth rates, etc.) , and/or to monitor udder health (e.g., as inflammation and/or infection can cause increased udder temperature, leading to an increase in milk from the impacted area of the udder). In such an example, in addition to generating data suitable for determining a characteristic(s) of the milk flowing through inline sensor 102, inline sensor can include a sensor(s) that can generate data suitable for monitoring operation of a particular milking device (e.g., a milking device of milking station 106-1 providing milk to milk line 104-1 that is analyzed by inline sensor 102-1), such as data that can be used to determine a volume of milk being received from the particular milking device, a flow rate of milk being received from the particular milking device, etc.

As another example, inline sensors for determining characteristics of milk are marketed by, and available from, Brolis Sensor Technology (headquartered in Vilnius, Lithuania), which can be used to accurately determine at least fat content, protein content, and lactose content of milk flowing through the sensor.

In some embodiments, multiple inline sensors (e.g., in different housings) can be used to measure different characteristics of milk flowing through a milk line and/or to monitor operation of a milking device from which the milk is received. For example, a first inline sensor on a milk line (e.g., an inline sensor 102-1a on milk line 104-1 with a portion 100-1 of system 100 as shown in FIG. 1B) can be used to generate data that can be used to determine a first subset of characteristics of the milk flowing through the milk line, and one or more additional inline sensors (e.g., another sensor(s) and/or located at different points along milk line 104-1), such as a second inline sensor on the milk line (e.g., an inline sensor 102-1b on milk line 104-1 as shown in FIG. 1B) can be used to generate data that can be used to determine a second subset of characteristics of the milk flowing through the milk line. As a more particular example, first inline sensor 102-1a can be used to generate image data and spectral data that can be used to determine an amount and/or concentration of components of milk flowing through milk line 104-1 (e.g., main components, ancillary and/or trace components, contaminants, etc.), and second inline sensor 102-1b can be used to generate electric data and temperature data that can be used to determine a parameter(s) indicative of an amount of milk that is flowing through (and/or has flowed through) second inline sensor 102-1b (e.g., a volumetric flow, a total volume, etc.) and/or an amount and/or concentration of a component(s) of milk flowing through inline sensor 102-1b (e.g., a number and/or concentration of somatic cells). Note that while first inline sensor 102-1a is shown upstream of second inline sensor 102-1b, various sensors can be placed in any suitable order. In such an example, there may be overlap between characteristics that can be determined from data generated by first inline sensor 102-1a and second inline sensor 102-1b, such as both first inline sensor 102-1a and second inline sensor 102-1b generating data that can be used to determine a flow rate of milk, a temperature of the milk, and/or a conductivity of the milk, or there may be no overlap between the first subset of characteristics and second subset of characteristics.

In a yet more particular example, first inline sensor 102-1a can be an inline sensor described in Buciunas et al. International Patent Application Publication No. WO2024/075079 (e.g., which can be used to determine an amount and/or concentration of various milk components based on data from a color sensor, a laser detector, and contact sensor(s), and a flow rate of milk through the inline sensor based on data from the contact sensor(s)), and second inline sensor 102-1b can be an inline sensor described in Hanes et al. U.S. Patent No. 10,598,528 (e.g., which can be used to determine at least a volumetric flow rate of milk through second inline sensor 102-1b based on data from contact sensor(s)).

In another yet more particular example, first inline sensor 102-1a can be a sensor from Brolis Sensor Technology, which can be used to determine at least fat content, protein content, lactose content, and temperature of the milk flowing through a milk line (e.g., milk line 104-1), and second inline sensor 102-1b can be a FloSmart sensor marketed by, and available from, BouMatic LLC (headquartered in Madison, Wisconsin, USA), which can be used to determine at least a volumetric flow of milk flowing through the milk line (e.g., milk line 104-1).

In some embodiments, inline sensor(s) 102 (and/or a particular type of inline sensor, if some milk lines are provided with multiple types of inline sensors) can be provided for a subset of N milk lines of M milking stations (and/or milk lines) in a dairy operation (e.g., dairy operation 120), and can be omitted from the other milk lines (e.g., the other M-N milk lines). For example, the subset of N milk lines which are provided with inline sensors 102 (e.g., inline sensors 102-1 to 102-N) can be a relatively small fraction of the M milking stations/milk lines (e.g., milking stations 106-1 to 106-M and/or milk lines 104-1 to 104-M) , which can depend on a target fraction of animals from which at least some minimum number of samples are to be collected within a predetermined period of time. In such an example, the subset of N milk lines can be a fraction of M that is expected to generate milk characteristics for at least a predetermined fraction, F, of cows within a predetermined period of time T (e.g., corresponding to less than a week, about a week, two weeks, a month, half of an average lactation period for the cow, an entire lactation period, etc.). In a more particular example, if each cow is milked an average of two times per day, and a target is set to collect samples from at least 90% of the cows (e.g., there is a 10% or less chance that any individual cow is not milked at a milking station equipped with an inline sensor) during each seven day period, if N inline sensors are distributed among the M milking stations such that each cow has an equal chance (e.g., N/M) of being milked at a milking station associated with an inline sensor, the minimum value of N can be at least 15% of M (e.g., N can be chosen such that *N* ≥ 0.15 * *M*), which can be expected to generate milk characteristics for at least 90% of the cows at least once within any seven day period. As the number of samples per animal within a predetermined period of time increases (e.g., if identification of relatively short-term trends is desirable) and/or the target chance of collecting at least one sample during the time period increases, the minimum number N also increases. Additionally, as the predetermined period of time increases (e.g., from seven days to fourteen days), the minimum number N can be expected to decrease. In some embodiments, if a sensor can measure milk characteristic(s) with high accuracy, measurements during a single milking session may be sufficient to determine accurate milk quality metrics for a particular animal during the milking session. For example, an inline sensor that meets current standards promoted by the International Committee for Animal Recording (ICAR) is expected to measure fat, protein, and lactose content for each milking to an accuracy of within a range of ±0.1% to ±0.5%.

In some embodiments, any suitable milk quality metrics can be generated based on data collected by an inline sensor(s). For example, data collected using an inline sensor across multiple milking session can be used to determine metrics that characterize a milk quality characteristic over a particular period of time, such as average fat content, average protein content, trends in udder health over time, trends in milk urea nitrogen (MUN) over time, milk yield consistency, and fat to protein rations.

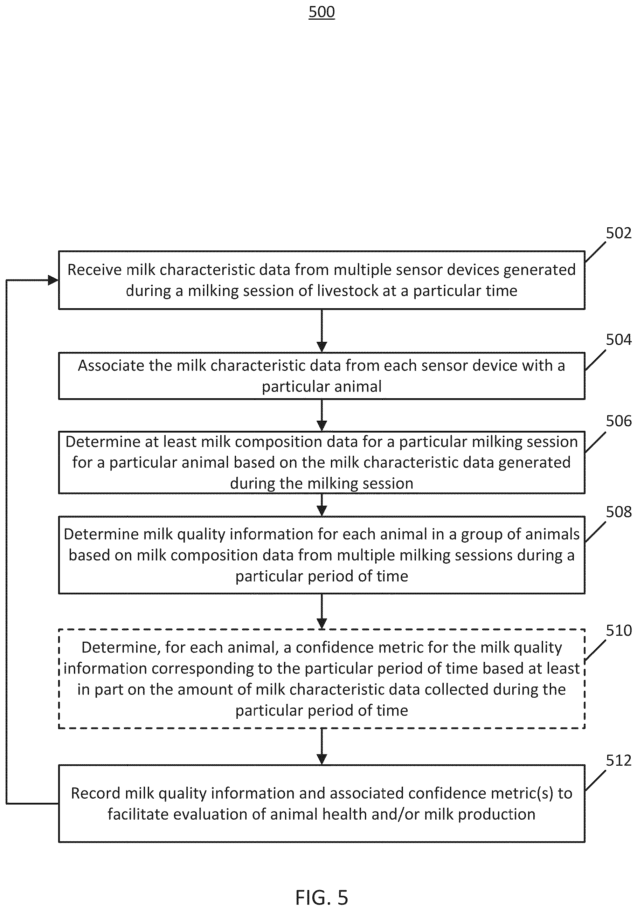

As described below in connection with FIG. 5, in some embodiments, milk characteristics from each cow can be monitored multiple times within a particular period of time (e.g., multiple times per week, per month, etc., as different cows are milked at different milking stations, some of which are provided with an inline sensor(s) and some which are not), and milk characteristic information for a particular cow over a predetermined period of time can be analyzed to calculate a confidence metric indicative of a reliability of the data, and/or determine that the data meets a reliability threshold (e.g., an ICAR standard). For example, milk characteristic information and an indication of reliability (e.g., an associated confidence metric, an indication that the milk characteristic information meets a predetermined reliability threshold) can inform a decision of a user of the milk characteristic information (e.g., an operator of the dairy, a veterinarian, etc.) of whether to adjust the care of the animal (e.g., adjusting an amount and/or composition of feed provided to the animal, adjusting whether and/or how often the animal is milked, whether to provide the animal with a particular treatment, etc.). As another example, milk characteristic information and an indication of reliability can inform an adjustment in automated system configured to provide care to the animal (e.g., an automated feed system, an automated milking system, etc.).

In some embodiments, milking stations 106 can be any suitable type of milking station. For example, milking stations 106 can be fully automated milking stations in which a robotic system prepares the cow for milking (e.g., including cleaning the teats) and attaches a milking device. As another example, milking stations 106 can be configured to facilitate machine milking in which a human operator prepares the cow for milking and attaches a milking device. Additionally, in some embodiments, milking stations 106 can be arranged in any suitable configuration. For example, milking stations 106 can be part of a rotary milking platform, a herringbone-style miking parlor, a tandem milking parlor, etc. Additionally or alternatively, milking stations 106 can include a mixture of different types of milking stations, and/or can be included in multiple different configurations.

In some embodiments, system 100 can include any suitable device or combination of devices that can be used to associate a particular animal with a milking station and/or inline sensor, which can be used to associate milk characteristic data and/or milk quality information with the animal which produced the milk. For example, system 100 can include one or more imaging devices can be used to capture image data of a milking station(s) and/or an animal associated with the milking station (e.g., as the animal is entering and/or exiting the milking station, as the animal is being milked at the milking station, etc.). As a more particular example, the image data can be analyzed to extract and/or read visually encoded data (e.g., alphanumeric text-based code such as an identification number printed on an ear tag or other surfaces, a machine-readable code such as a QR code affixed to the animal, etc.). As another more particular example, the image data can be analyzed to identify a particular animal based on the visual appearance of the animal in the image data (e.g., based on facial and/or other characteristics of the animal).

As another example, system 100 can include one or more wireless communication devices can be used to read information encoded in a device affixed to the animal. As a more particular example, one or more radio frequency identification (RFID) readers, near field communication (NFC) readers, ultrawideband (UWB) devices, Bluetooth devices, and/or any other suitable type of wireless communication technology that can be used for wireless identification, can be used to read identifying information encoded in a passive or active transmitter device affixed to the animal (e.g., as part of an ear tag, attached via a collar, implanted subcutaneously, etc.), and based on the location of the transmitter device, can associate a particular animal with a particular milking station. In such an example, the wireless communication device can be a fixed part of system 100, or a mobile device (e.g., a general purpose device such as a smartphone or wearable computing device, or a special purpose device configured to read data from a nearby transmitter affixed to an animal).

As yet another example, system 100 can receive input associating a particular animal (e.g., via a text-based code, an optical code, etc.) with a particular milking station. In a more particular example, such input can be received from a mobile device (e.g., a general purpose device such as a smartphone or wearable computing device executing a frontend of a milk quality analysis system, or a special purpose device configured to identify a milking station and an animal positioned at the milking station), or a fixed device (e.g., a keypad or touchscreen installed near a milking station). Additional particular examples of systems and devices that can be used to associate a particular animal with a milking station are described in Rajkondawar et al. U.S. Patent No. 8,950,357, Siddell U.S. Patent No. 8,955,459, and Hofman et al. U.S. Patent No. 11,096,370.

In some embodiments, system 100 can include any suitable number of activity monitoring sensors 122, can include a device(s) that is configured to measure and/or determine a position, posture, activity, etc., of one or more animals. In some embodiments, activity monitoring sensors 122 can include one or more devices to determine one or more activity metrics that can be used to estimate metabolic information of an animal, such as an amount of time standing, lying, walking, feeding, watering, an amount of calories ingested, an amount of calories expended (e.g., through milk production, basal metabolic activity, movement, etc.), an amount of one or more macronutrients consumed, etc. In some embodiments, each animal can be associated with a particular activity monitoring sensor 122 affixed to the animal, which can be configured to measure one or more values indicative of activity. For example, a device that includes components (e.g., one or more active sensors, one or more passive sensors, receivers, transceivers, etc.) configured to measure location (e.g., using a satellite-based radio navigation system such as GPS, a local positioning system based on radio waves or acoustic waves, etc.) and/or motion data (e.g., using one or more accelerometers, gyroscopes, etc.) can be affixed to each animal, and can wirelessly transmit collected data (e.g., to a device used to implement livestock management system 112). As another example, a device that includes components (e.g., one or more transmitters, transceivers, etc.) configured to facilitate location measurement (e.g., using a remote monitoring system based on radio waves) can be affixed to each animal, and a remote device(s) can determine the location. As a particular example, activity monitoring sensors 122 can include devices described in Rajkondawar et al. U.S. Patent No. 8,949,034 that are configured to measure and/or determine that position, posture, and activity of cows in a monitored area. As another particular example, activity monitoring sensors 122 can include devices affixed to particular cows that are configured to measure and/or determine the position, posture, and activity of cows (e.g., a monitoring device affixed with a collar).

In some embodiments, system 100 can include a livestock management system 112, which can be used to store information related to operation of dairy operation 120 and/or information related to livestock animals, such as cows milked at dairy operation 120. For example, livestock management system 112 can receive data from one or more sensors (e.g., inline sensors 102, activity monitoring sensors such as sensors 122), devices, and/or systems (e.g., a system that associates a particular cow with a particular milking station), and/or input from one or more devices (e.g., input provided by an operator of dairy operation 120 via a user interface, such as a graphical user interface presented by a computing device). In a more particular example, livestock management system 112 can receive data indicative of one or more characteristics of milk flowed through an inline sensor (e.g., 102) during milking, and associate the data with the particular animal that produced the milk based on information indicating which animal was being milked at the milking station associated with the inline sensor during the time the data was generated. As another example, livestock management system 112 can receive activity data (e.g., from sensor(s) 122) indicative of one or more activities performed by a particular animal, and associate the activity data with the particular animal (e.g., based on identification information associated with the activity data and/or associated with a device that transmitted the data).

Additionally, in some embodiments, livestock management system 112 can use received and/or stored data to determine any suitable information about a particular animal, which can be used to monitor and/or evaluate an animal. For example, livestock management system 112 can use received data to determine milk volume information, milk composition information, and/or any other suitable information that can be used in evaluating the production and/or health of a particular animal (e.g., a particular cow). In some embodiments, livestock management system 112 can be implemented locally and/or remotely. For example, livestock management system 112 can be implemented using only local devices within dairy operation 120 (e.g., sensors 102, local computing devices that receive and/or aggregate data from sensors 102, etc.). As another example, livestock management system 112 can be implemented using local devices within dairy operation 120 (e.g., sensors 102, local computing devices that receive and/or aggregate data from sensors 102, etc.) and remote devices (e.g., a remote server(s), a cloud computing service, etc.). As yet another example, livestock management system 112 can be implemented using primarily remote devices (e.g., a remote server(s), a cloud computing service, etc., that receives raw data from local sensor devices, such as inline sensors 102).

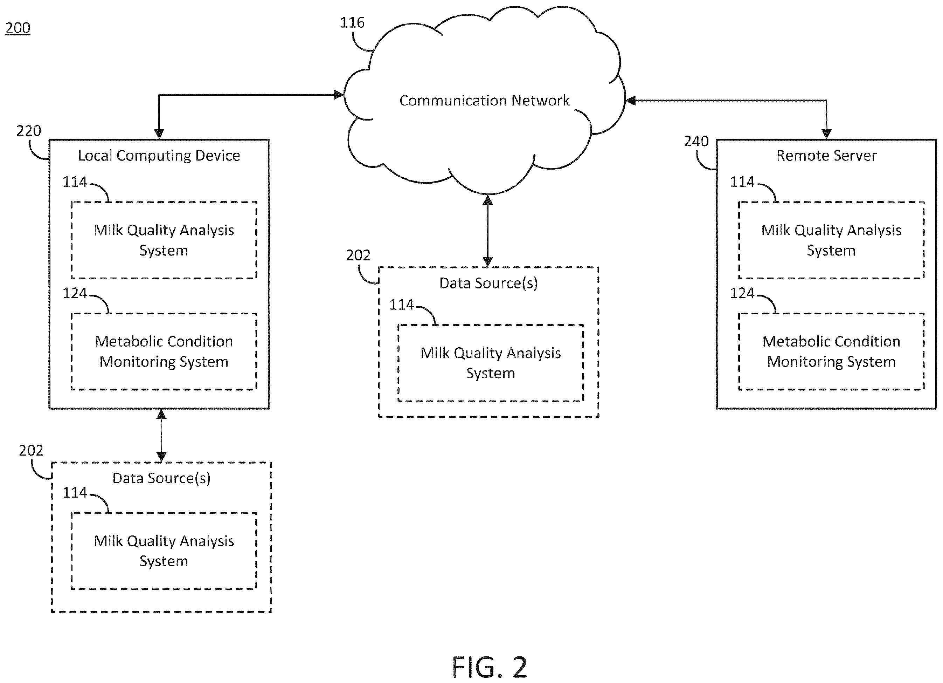

In some embodiments, system 100 can include a milk quality analysis system 114, which can be used to determine one or more milk quality metrics for each animal that has been milked at a milking station associated with a suitable inline sensor (e.g., milking station 106-1 and inline sensor 102-1) based on data generated by and/or received from the inline sensor. As described below, in some embodiments, milk quality analysis system 114 can be at least partially implemented in livestock management system 112 on a remote server (e.g., as a cloud application, within a software as a service platform, etc.). Additionally or alternatively, in some embodiments, milk quality analysis system 114 can be at least partially implemented in devices within dairy operation, such as sensors 102, a local computing device, etc. For example, as described below in connection with FIG. 2, milk quality analysis system 114 can be implemented as a distributed application across multiple devices, such as within a data source (e.g., inline sensor 102), within a local computing device (e.g., a mobile computing device such as a smartphone, tablet computer, etc., a laptop computer, a desktop computer, a local server, etc.) in communication with the data source, and/or within a remote computing device (e.g., a dedicated remote server, a device within a cloud computing system). In such an example, milk quality analysis system 114 can generate milk quality data and/or any other suitable data from raw sensor outputs (e.g., within inline sensor 102, at a local computing device, and/or at a remote computing device), aggregate milk quality data (e.g., within inline sensor 102, at a local computing device, and/or at a remote computing device), record milk quality data in connection with a particular animal (e.g., at a local computing device, and/or at a remote computing device), calculate additional milk quality data for a particular animal (e.g., over time) and/or for multiple animals (e.g., a particular herd) over any suitable period of time (e.g., at a local computing device, and/or at a remote computing device).

In some embodiments, system 100 can include a metabolic condition monitoring system 124, which can be used to determine one or more metabolic condition metrics for each animal for which activity and/or other suitable data is available based on data generated by and/or received from activity monitoring sensors 122 and/or data generated by and/or received from inline sensors 102. As described below, in some embodiments, metabolic condition monitoring system 124 can be at least partially implemented in livestock management system 112 on a remote server (e.g., as a cloud application, within a software as a service platform, etc.). Additionally or alternatively, in some embodiments, metabolic condition monitoring system 124 can be at least partially implemented in devices within dairy operation 120, such as inline sensors 102, activity monitoring sensors 122, a local computing device, etc. For example, as described below in connection with FIG. 2, metabolic condition monitoring system 124 can be implemented as a distributed application across multiple devices, such as within a data source (e.g., activity monitoring sensor 122, inline sensor 102), within a local computing device (e.g., a mobile computing device such as a smartphone, tablet computer, etc., a laptop computer, a desktop computer, a local server, etc.) in communication with the data source, and/or within a remote computing device (e.g., a dedicated remote server, a device within a cloud computing system). In such an example, metabolic condition monitoring system 124 can generate metabolic condition metrics and/or any other suitable data from raw sensor outputs (e.g., within activity monitoring sensor(s) 122, at a local computing device, and/or at a remote computing device), aggregate metabolic data and/or metrics (e.g., within activity monitoring sensor(s) 122, at a local computing device, and/or at a remote computing device), record metabolic data and/or metrics in connection with a particular animal (e.g., at a local computing device, and/or at a remote computing device), calculate additional metabolic data for a particular animal (e.g., over time) and/or for multiple animals (e.g., a particular herd) over any suitable period of time (e.g., at a local computing device, and/or at a remote computing device).

In some embodiments, data generated by a sensor(s) (e.g., inline sensors 102, activity monitoring sensor(s) 122) can be transmitted to a computing device (e.g., a local computing device, a remote computing device, etc.) via a communication network 116, which can be any suitable communication network or combination of communication networks. For example, communication network 116 can include a Wi-Fi network (which can include one or more wireless routers, one or more switches, etc.), a peer-to-peer network (e.g., a Bluetooth network), a cellular network (e.g., a 3G network, a 4G network, a 5G network, etc., complying with any suitable standard(s), such as CDMA, GSM, LTE, LTE Advanced, 5G NR, etc.), a wired network, etc. In some embodiments, communication network 116 can include one or more portions of a local area network (LAN), a wide area network (WAN), a public network (e.g., the Internet, which may be part of a WAN and/or LAN), any other suitable type of network, or any suitable combination of networks. Communications links shown in FIGS. 1 to 3 can each be any suitable communications link or combination of communications links, such as wired links, fiber optic links, Wi-Fi links, Bluetooth links, cellular links, etc.

In some embodiments, system 100 can include farm automation equipment 118, which can include any suitable devices configured to automate one or more tasks associated with dairy operation 120 and/or collect data related to dairy operation 120 and/or animals associated with dairy operation 120. For example, farm automation equipment 118 can include an automated feeding system (e.g., a robotic feeding system) that uses one or more devices to automatically dispense feed (e.g., a particular quantity and/or composition of feed) to be consumed by one or more particular animals at least partially without human intervention. In such an example, the amount and/or composition of the feed can be based on data stored in livestock management system 112, such as data related to health information, milk quality information, and/or milk production information associated with the animal for which the feed is being dispensed. As another example, farm automation equipment 118 can include an automated milking system (e.g., a robotic milking system) that uses one or more devices to automatically milk cows at least partially without human intervention.

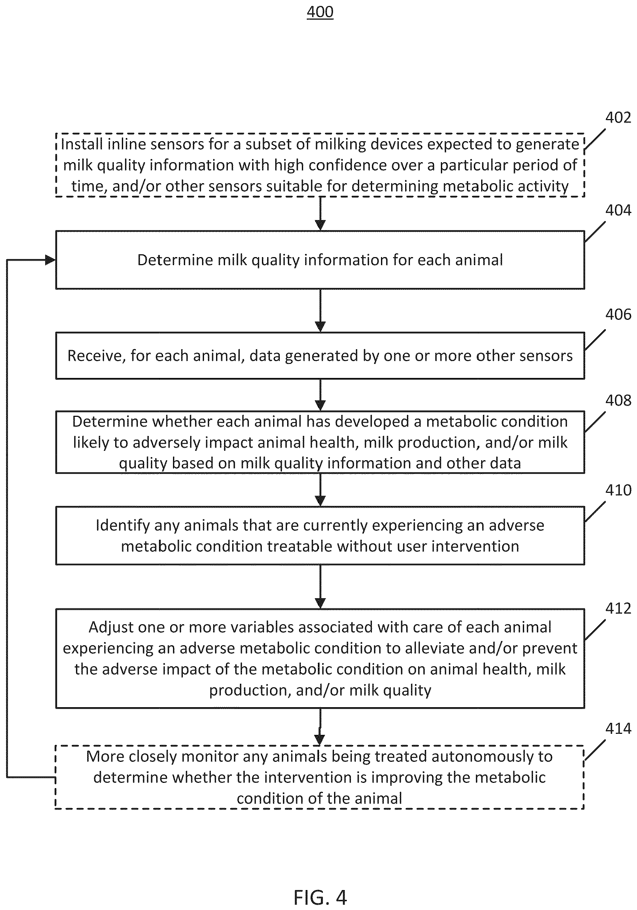

In some embodiments, farm automation equipment 118 can be controlled to operate on a predetermined schedule, in response to sensor data (e.g., in response to determining that a particular condition has been satisfied, such as that a particular animal has entered a feeding area) measured by and/or received by farm automation equipment 118, and/or based on instructions from a computing device (e.g., a computing device implementing livestock management system 112). For example, in some embodiments, farm automation equipment 118 can receive instructions from livestock management system 112 to dispense a particular amount of one or more types of feed and/or supplements (e.g., to treat and/or prevent one or more conditions that adversely impact the health and/or milk production of the cow) to a particular cow, and can autonomously dispense the instructed type(s) feed and/or supplements in the instructed amount to the cow based on a location of the cow. As a more particular example, as described below in connection with FIGS. 4 to 7, livestock management system 112 can instruct farm automation equipment 118 to add a supplement (e.g., a glycol-based supplement, such as a propylene glycol supplement).

In some embodiments, farm automation equipment 118 can be used to monitor certain animal activity (e.g., in addition to, or in lieu of, activity monitoring sensor(s) 122) using any suitable sensor(s). For example, farm automation equipment 118 can monitor feed consumption for each animal using any suitable sensor(s) and/or combination of sensors. In a more particular example, farm automation equipment 118 can use one or more imaging devices (e.g., for capturing digital images, video, 3D scene depth information, etc.) to determine how much feed has been consumed by a particular animal. In a yet more particular example, farm automation equipment 118 can use one or more computer vision techniques (e.g., which may or may not incorporate machine learning techniques) to estimate an amount of feed dispensed to the animal (which may also be determined based on an amount of feed that a robotic feeding system was instructed to dispense to the animal and/or an amount of feed that a robotic feeding system reported dispensing to the animal), and/or to estimate an amount of feed consumed by the animal (e.g., based on an estimate of how much feed is remaining and an amount of feed that was dispensed by the animal). In another more particular example, farm automation equipment 118 can use one or weighing devices (e.g., a scale) to determine how much feed has been consumed by a particular animal (e.g., based on a weight of feed that was provided and a weight of feed that remained when the animal finished feeding).

FIG. 2 shows an example of a system 200 for monitoring the metabolic condition of dairy livestock using milk quality data from inline sensors in accordance with some embodiments of the disclosed subject matter. As shown in FIG. 2, a local computing device 220 can receive data from a data source 202. In some embodiments, local computing device 220 can execute at least a portion of milk quality analysis system 114 to determine milk quality information based on data generated by, and/or received from, data source 202, and/or execute at least a portion of metabolic condition monitoring system 124 to determine metabolic condition information based on data generated by, and/or received from, data source 202. Additionally or alternatively, in some embodiments, local computing device 220 can communicate data received from data source(s) 202 to a server 240 over a communication network 116 and/or server 240 can receive data from data source 202 (e.g., directly and/or indirectly using communication network 116), which can execute at least a portion of milk quality analysis system 114 and/or metabolic condition monitoring system 124. In such embodiments, server 240 can return information to local computing device 220 (and/or any other suitable computing device), such as milk quality information stored by and/or determined by milk quality analysis system 114, and/or metabolic condition information stored by and/or determined by metabolic condition monitoring system 124. In some embodiments, milk quality analysis system 114 can execute one or more portions of process 400 described below in connection with FIG. 4, process 500 described below in connection with FIG. 5, and/or any other suitable processes.

In some embodiments, local computing device 220 and/or server 240 can be any suitable computing device or combination of devices, such as a desktop computer, a laptop computer, a smartphone, a tablet computer, a wearable computer, a server computer, a virtual machine being executed by a physical computing device, etc.

In some embodiments, data source(s) 202 can be any suitable source(s) of data that can be used to determine milk quality information and/or metabolic condition information for a particular dairy livestock (e.g., a cow) or a group of dairy livestock (e.g., a herd or a portion of a herd) as described herein. For example, data source(s) 202 can include an inline sensor(s) (e.g., one or more of inline sensors 102 described above in connection with FIGS. 1A and 1B). As another example, data source(s) 202 can include a device(s) that is configured to measures and/or determine a position, posture, activity, etc., of one or more animals. In a more particular example, data source(s) 202 can include devices described in Rajkondawar et al. U.S. Patent No. 8,949,034 that are configured to measure and/or determine that position, posture, and activity of cows in a monitored area. As another more particular example, data source(s) 202 can include devices affixed to particular cows that are configured to that are configured to measure and/or determine the position, posture, and activity of cows (e.g., a monitoring device affixed with a collar).

In some embodiments, one or more data sources 202 can be in direct communication with local computing device 220 (e.g., local computing device 220 can be located within the same building as inline sensors 102). For example, data source 202 can communicate with local computing device 220 via a direct wired connection or a direct wireless connection (e.g., a peer-to-peer connection). Additionally or alternatively, in some embodiments, one or more data sources 202 can be located locally to, and/or remotely from, local computing device 220, and can communicate data (e.g., data generated by an inline sensor or other data source) and/or information (e.g., information calculated by an inline sensor or other data source) to local computing device 220 (and/or server 240) via a communication network (e.g., communication network 116).

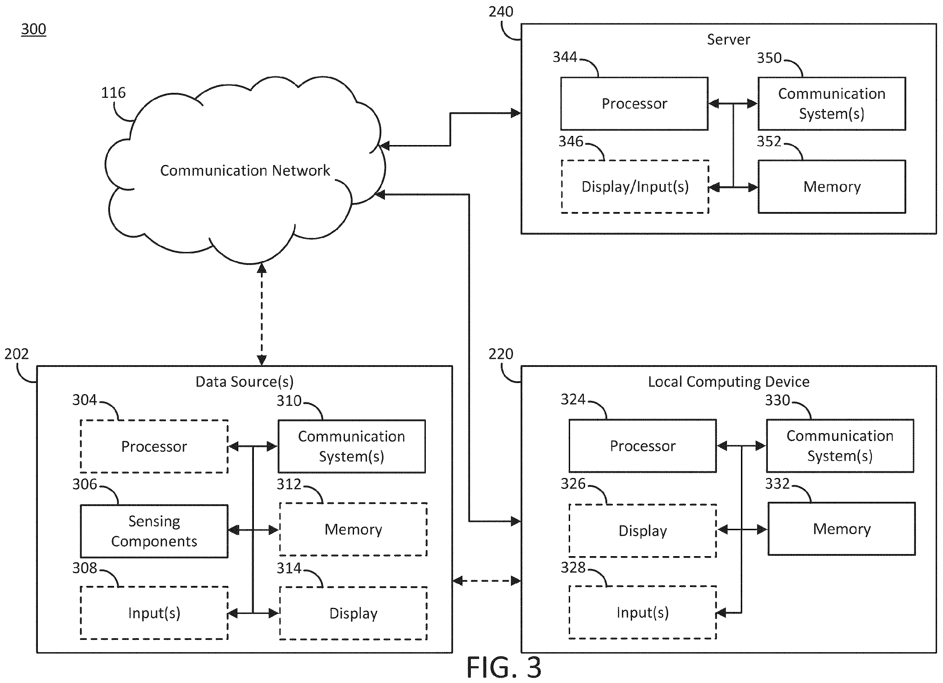

FIG. 3 shows an example of hardware 300 that can be used to implement data source 202, computing device 220, and server 240 of FIG. 2 in accordance with some embodiments of the disclosed subject matter. As shown in FIG. 3, in some embodiments, data source(s) 202 can include a processor 304, sensing components 306, one or more inputs 308, one or more communication systems 310, memory 312, and/or a display(s) 314. In some embodiments, processor 304 can be any suitable hardware processor or combination of processors, such as a central processing unit (CPU), an accelerated processing unit (APU), a graphics processing unit (GPU), a field programmable gate array (FPGA), an application specific integrated circuit (ASIC), a microcontroller, etc. In some embodiments, sensing components 306 can include components that can be used to generate data that can be used to monitor milk quality of dairy livestock, such as one or more optical components, one or more contact sensors, etc. that can be used to measure properties of milk passing through an inline sensor. Additionally or alternatively, in some embodiments, sensing components 306 can include components that can be used to generate data that can be used to monitor the position, posture, activity, etc., of dairy livestock, such as one or more electrical sensors (e.g., an RFID reader, a NFC reader, an UWB transceiver, a Bluetooth transceiver, a global navigation satellite system (GNSS) receiver such as a global position system (GPS receiver), etc.), one or more imaging devices (e.g., one or more digital cameras, thermal cameras, etc.), one or more inertial measurement devices (e.g., one or more accelerometers, gyroscopes, etc.), etc. In some embodiments, inputs 308 can include any suitable input devices and/or sensors that can be used to receive user input, such as a keyboard, a mouse, a touchscreen, a touchpad, a microphone, a camera, etc. In some embodiments, display 314 can include any suitable display devices, such as a touchscreen, a computer monitor, a television, etc. In some embodiments, data source(s) 202 can omit processor 304 and/or memory 312 (e.g., where data source 202 is an inline sensor configured to stream raw data to local computing device 220 device or server 204). Additionally, in some embodiments, data source(s) 202 can omit inputs 308, and/or a display(s) 314 (e.g., where data source 202 that is not configured for direct user interaction). For example, data source 202 can provide data, results of an analysis, etc., to local computing device 220 and/or server 240, and local computing device 220 and/or server 240 can use the data, results, and/or a user interface to execute at least a portion of a process(es) described herein, present metabolic condition information, milk quality information, present a user interface, etc.

In some embodiments, sensing components 306 can include components that are used to measure data indicative of milk quality and/or animal health. For example, in some embodiments, data source 202 can include sensing components used to implement an inline sensor(s) (e.g., one or more of inline sensors 102 described above in connection with FIGS. 1A and 1B). As another example, in some embodiments, data source 202 can include sensing components used to implement an activity monitoring sensor(s) (e.g., one or more activity monitoring sensor(s) 122 described above in connection with FIG. 1A). As yet another example, in some embodiments, data source 202 can include sensing components used to implement a mobile computing device, such as an internet-of-things (IoT) device, a smartphone, a tablet computer, a laptop computer, a wearable computing device, etc.

In some embodiments, communication system(s) 310 can include any suitable hardware, firmware, and/or software for communicating information over a communication network 116 and/or any other suitable communication networks. For example, communication systems 310 can include one or more transceivers, one or more communication chips and/or chip sets, etc., that can be used to establish a wired and/or wireless communication link. In a more particular example, communication systems 310 can include hardware, firmware, and/or software that can be used to establish a direct or indirect wired connection and/or a direct or indirect wireless connection, such as a Bluetooth connection, Bluetooth Low Energy connection, an UWB connection, an NFC connection, a ZigBee connection, a Wi-Fi connection, a cellular connection (e.g., an uplink connection, a downlink connection, or a sidelink connection), an Ethernet connection, etc.

In some embodiments, memory 312 can include any suitable storage device or devices that can be used to store instructions, values, etc., that can be used, for example, by processor 304 to perform processes described herein, to capture data, to store data, to retrieve data, etc., to communicate with local computing device 220 and/or server 240 via communication system(s) 310, etc. Memory 312 can include any suitable volatile memory, non-volatile memory, storage, or any suitable combination thereof. For example, memory 312 can include random access memory (RAM), read-only memory (ROM), electronically erasable programmable read-only memory (EEPROM), one or more flash drives, one or more hard disks, one or more solid state drives, one or more optical drives, etc.

In some embodiments, memory 312 can have encoded thereon a computer program for controlling operation of data source 202. In such embodiments, processor 304 can execute at least a portion of the computer program to: generate raw measurement data, determine activity data (e.g., based on location data, acceleration data, etc.), generate one or more metabolic condition metrics, determine milk characteristic data (e.g., based on the raw data), generate one or more milk quality metrics, store milk characteristic data and/or milk quality metrics in memory 312, retrieve milk characteristic data and/or milk quality metrics from storage in memory 312, transmit information (e.g., activity data, metabolic condition metrics, milk characteristic data, and/or milk quality metrics) to local computing device 220 and/or server 240, to execute at least a portion of a process for monitoring metabolic condition of one or more dairy livestock, such as one or more portions of processes described below in connection with FIGS. 4 to 6, etc.

In some embodiments, local computing device 220 can include a processor 324, a display 326, one or more inputs 328, one or more communication systems 330, and/or memory 332. In some embodiments, processor 324 can be any suitable hardware processor or combination of processors, such as a CPU, an APU, a GPU, an FPGA, an ASIC, a microcontroller, etc. In some embodiments, display 326 can include any suitable display devices, such as a computer monitor, a touchscreen, a television, etc. In some embodiments, inputs 328 can include any suitable input devices and/or sensors that can be used to receive user input, such as a keyboard, a mouse, a touchscreen, a microphone, etc. In some embodiments, local computing device 220 can omit inputs (e.g., where local computing device 220 is a backend device that is not configured for direct user interaction). For example, local computing device 220 can provide results of an analysis, raw measurement data, activity data, metabolic condition metrics, milk characteristic data, and/or milk quality metrics, and/or a portion of a user interface to another computing device (e.g., a frontend device configured for direct user interaction), which can use the data, metrics, etc., and/or user interface to execute at least a portion of a process(es) described herein, present data and/or information, present a user interface, etc. In some embodiments, any suitable computing device (e.g., a desktop computer, a server computer, a laptop computer, a smartphone, a tablet computer, a wearable computer, a virtual machine being executed by a physical computing device, etc.) can be used to implement local computing device 220.

In some embodiments, communication systems 330 can include any suitable hardware, firmware, and/or software for communicating information over communication network 116 and/or any other suitable communication networks. For example, communication systems 330 can include one or more transceivers, one or more communication chips and/or chip sets, etc., that can be used to establish a wired and/or wireless communication link. In a more particular example, communication systems 330 can include hardware, firmware, and/or software that can be used to establish a direct or indirect wired connection and/or a direct or indirect wireless connection, such as a Bluetooth connection, a Bluetooth Low Energy connection, an UWB connection, an NFC connection, a ZigBee connection, a Wi-Fi connection, a cellular connection (e.g., an uplink connection, a downlink connection, or a sidelink connection), an Ethernet connection, etc.

In some embodiments, memory 332 can include any suitable storage device or devices that can be used to store instructions, values, etc., that can be used, for example, by processor 324 to communicate with data source 202 and/or server 240 via communication system(s) 330, receive data from data source device(s) 202, receive additional data and/or instructions via inputs 328 and/or from a remote computing device (e.g., server 240), determine activity data and/or metabolic condition metrics, analyze activity data and/or metabolic condition metrics associated with a dairy livestock or group of dairy livestock, determine milk characteristic data and/or milk quality metrics, analyze milk characteristic data and/or milk quality metrics associated with a dairy livestock or group of dairy livestock, provide data and/or metrics (e.g., activity data, metabolic condition metrics, milk characteristic data, milk quality metrics, etc.) to a remote computing device for analysis (e.g., server 240), to present a user interface that includes metabolic condition and/or milk quality information, etc. Memory 332 can include any suitable volatile memory, non-volatile memory, storage, or any suitable combination thereof. For example, memory 332 can include RAM, ROM, EEPROM, one or more flash drives, one or more hard disks, one or more solid state drives, one or more optical drives, etc.

In some embodiments, memory 332 can have encoded thereon a computer program for controlling operation of local computing device 220. In such embodiments, processor 324 can use the computer program to: receive raw measurement data (e.g., from data source(s) 202), determine activity data (e.g., based on location data, acceleration data, etc.), receive activity data (e.g., from data source(s) 202), generate one or more metabolic condition metrics, determine milk characteristic data (e.g., based on the raw data), receive milk characteristic data (e.g., from data source(s) 202), generate one or more milk quality metrics (e.g., based on milk characteristic data), store milk characteristic data and/or milk quality metrics in memory 332, retrieve data (e.g., activity data, metabolic condition metrics, milk characteristic data, milk quality metrics, etc.) from storage in memory 332, transmit information (e.g., activity data, metabolic condition metrics, milk characteristic data, milk quality metrics, etc.) to another local computing device and/or server 240, to execute at least a portion of a process for monitoring metabolic condition of one or more livestock, such as one or more portions of processes described below in connection with FIGS. 4 to 6, etc.

In some embodiments, server 240 can include a processor 344, a display and/or input(s) 346, a communication system(s) 350, and/or memory 352. In some embodiments, processor 344 can be any suitable hardware processor or combination of processors, such as a CPU, an APU, a GPU, an FPGA, an ASIC, a microcontroller, etc.

In some embodiments, display and/or input(s) 346 can include any suitable display devices, such as a computer monitor, a touchscreen, a television, etc., and/or can include any suitable input devices and/or sensors that can be used to receive user input, such as a keyboard, a mouse, a touchscreen, a microphone, etc. In some embodiments, server 240 can omit display/input(s) 346 (e.g., where server 240 is not configured for direct user interaction). For example, server 240 can communicate with another computing device (e.g., local computing device 220, data source 202, etc.) via communication system(s) 350 to: receive raw measurement data (e.g., from data source(s) 202 and/or local computing device 220); receive activity data (e.g., from data source(s) 202 and/or local computing device 220); receive one or more metabolic condition metrics; receive milk characteristic data (e.g., from data source(s) 202); receive one or more milk quality metrics; transmit information (e.g., activity data, metabolic condition metrics, milk characteristic data, milk quality metrics, etc.) to local computing device 220, another server, and/or a different user computer device (e.g., which may or may not be located in or near dairy operation 120); provide data and/or a portion of a user interface to be used to present metabolic condition information and/or to present a user interface that includes metabolic condition information and/or milk quality information; instruct (e.g., without user intervention) automated equipment (e.g., automated farm equipment 118) to adjust a variable(s) associated with care of an animal based on an analysis of metabolic condition information and/or milk quality information; etc.

In some embodiments, communication systems 350 can include any suitable hardware, firmware, and/or software for communicating information over communication network 116 and/or any other suitable communication networks. For example, communication systems 350 can include one or more transceivers, one or more communication chips and/or chip sets, etc., that can be used to establish a wired and/or wireless communication link. In a more particular example, communication systems 350 can include hardware, firmware, and/or software that can be used to establish a direct or indirect wired connection and/or a direct or indirect wireless connection, such as a CAN bus connection, a Bluetooth connection, Bluetooth Low Energy connection, an UWB connection, an NFC connection, a ZigBee connection, a Wi-Fi connection, a cellular connection (e.g., an uplink connection, a downlink connection, or a sidelink connection), an Ethernet connection, etc.

In some embodiments, memory 352 can include any suitable storage device or devices that can be used to store instructions, values, etc., that can be used, for example, by processor 344 to communicate with data source 202, local computing device 220, etc., via communication system(s) 350, etc. Memory 352 can include any suitable volatile memory, non-volatile memory, storage, or any suitable combination thereof. For example, memory 352 can include RAM, ROM, EEPROM, one or more flash drives, one or more hard disks, one or more solid state drives, one or more optical drives, etc.

In some embodiments, memory 352 can have encoded thereon a computer program for controlling operation of server 240. In such embodiments, processor 344 can use the computer program to: receive raw measurement data (e.g., from data source(s) 202), determine activity data (e.g., based on location data, acceleration data, etc.), receive activity data (e.g., from data source(s) 202), generate one or more metabolic condition metrics, determine milk characteristic data (e.g., based on the raw data), receive milk characteristic data (e.g., from data source(s) 202 and/or local computing device 220), generate one or more milk quality metrics (e.g., based on milk characteristic data), store milk characteristic data and/or milk quality metrics in memory 352, retrieve data (e.g., activity data, metabolic condition metrics, milk characteristic data, milk quality metrics, etc.) from storage in memory 352, transmit information (e.g., activity data, metabolic condition metrics, milk characteristic data and/or milk quality metrics) to local computing device 220, another server, and/or a user computing device (e.g., a smartphone, tablet computer, laptop computer, desktop computer, wearable computer, etc.), to execute at least a portion of a process for monitoring metabolic condition of one or more livestock, such as one or more portions of processes described below in connection with FIGS. 4 to 6, etc.

FIG. 4 shows an example of a process 400 for automated metabolic condition monitoring and treatment of dairy livestock using inline sensors in accordance with some embodiments of the disclosed subject matter.

At 402, process 400 can include installing inline sensors (e.g., an inline sensor(s) described above in connection with FIGS. 1A and 1B) for a subset of milking devices that is expected to generate milk quality information with a high confidence over a particular period of time. In some embodiments, inline sensors installed at 402 can be incorporated into milking equipment (e.g., can be integrated into a milk line), or can be separate from milking equipment that has already been installed and/or is being installed. For example, inline sensors installed at 402 can be added to an existing milk line.

In some embodiments, the number of inline sensors installed at 402 can be at least a minimum number such that reliable (e.g., high confidence) information about the milk quality of all dairy livestock (e.g., cows) milked at a particular milking operation over a predetermined period of time. As described above, as the predetermined time period decreases, the ratio of inline sensors to milking stations may need to be increased to generate reliable data for all cows over the predetermined time period that can be used to inform decisions for each cow. For example, a relatively small fraction of milk lines (e.g., about 10%) can be equipped with inline sensors to generate reliable milk quality information for each cow in a herd over longer periods of time (e.g., multiple weeks, a month, or more). As the period of time over which reliable data is desired increases, the fraction of milk lines that need to be equipped with inline sensors generally decreases. In such an example, milk quality data for each cow can be used to inform decisions about breeding selection for increasing desirable traits and/or avoiding undesirable traits. As a more particular example, to reliably collect samples from at least 90% of cows in a herd every week when milking twice per day, about 15% of milking stations can be equipped with inline sensors. As another example, a larger fraction of milk lines (e.g., about 30% or more) can be equipped with inline sensors to generate reliable milk quality information for each cow in a herd over a shorter period of time (e.g., less than a week or multiple times per week). In such an example, milk quality data for each cow can be used to monitor health of cows that are being milked and inform actions to mitigate health conditions that are difficult to detect and treat economically using conventional laboratory testing and/or routine veterinary care. As a more particular example, to reliably collect samples from at least 90% of cows in a herd every three to four days when milking about twice per day, about 30% of milking stations can be equipped with inline sensors. As another more particular example, to reliably collect two samples from at least 90% of cows in a herd twice per week when milking about twice per day, about 40% of milking stations can be equipped with inline sensors. As yet another more particular example, to reliably detect that a cow that is experiencing a subclinical negative energy balance prior to the progressing to a clinical energy imbalance based at least in part on milk quality information, the fraction of milk lines equipped with inline sensors can be a fraction that is expected to generate reliable milk quality information for each cow in a herd within a relatively short period of time (e.g., reliably detecting subclinical NEB in any individual cow prior to progression may require that all, or nearly all, milk lines be monitored. In some embodiments, incidence of a metabolic condition, such as NEB, in a herd can be estimated based on testing of a statistically significant sample of animals (e.g., at least twelve animals at once). Note that sensors may be installed in groups of predetermined size, and the number of milk lines being monitored may exceed the minimum number. For example, if inline sensors are installed in sets of eight, a milking parlor with 40 to 50 milking stations can be equipped with inline sensors (e.g., inline sensors 102) on eight milk lines if the target fraction of lines is 15%, sixteen (two sets of eight) milk lines if the target fraction of lines is 30%, and sixteen or twenty four (three sets of eight) milk lines if the target fraction of lines is 40% (e.g., depending on the total number of milking stations).

In some embodiments, if a relatively small fraction of milk lines of a dairy operation is already equipped with inline sensors (e.g., inline sensors 102) used to monitor milk quality information over a longer period of time, additional inline sensors can be installed at 402. Alternatively, if a sufficiently large fraction of milk lines of a dairy operation is already equipped with inline sensors used to monitor milk quality, 402 can be omitted from process 400.

At 404, process 400 can determine milk quality information for each animal of a group of animals (e.g., a herd of cows) from which milk is collected using any suitable technique or combination of techniques. For example, process 400 can determine milk quality information using techniques described below in connection with process 500 of FIG. 5.

At 406, process 400 can receive, for at least animals in the group of animals, data generated from one or more other sensors that can be used to determine metabolic information of the animal. For example, in some embodiments, process 400 can receive data indicative of how active each animal was over a particular period of time and/or that characterizes which activities the animal engaged in during the particular period of time. As a more particular example, process 400 can receive activity data from one or more activity monitoring sensors (e.g., activity monitoring sensors 122 described above in connection with FIG. 1A).

In some embodiments, the data received at process 400 can include data that is useable to determine any suitable activity metrics and/or metabolic condition metrics, such as metrics described above in connection with FIG. 1A (e.g., time standing, lying, walking, feeding, watering, etc., calories ingested, calories expended, etc.). For example, in some embodiments, a decrease in time eating, time ruminating, and/or time active can be an indicator that a cow may be experiencing an NEB state.

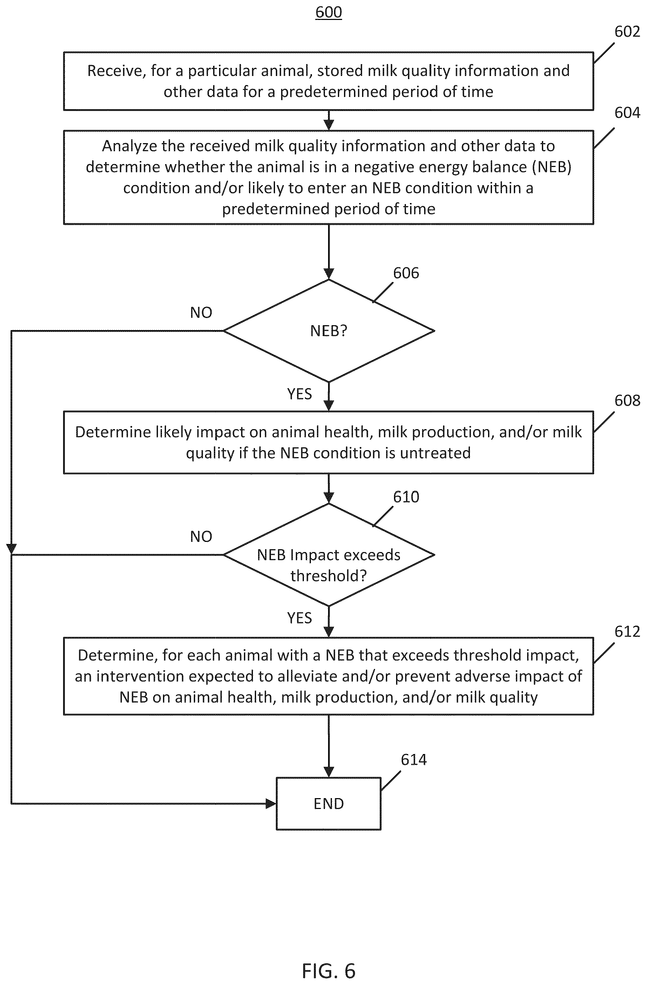

At 408, process 400 can determine whether each animal has developed a metabolic condition that is likely to adversely impact animal health, milk production, and/or milk quality based on milk quality information and other data. In some embodiments, process 400 can use the milk quality information and other data (e.g., milk temperature, milk production, monitored activity, eating time, rumination time, etc.) to evaluate each animal for one or more metabolic conditions (e.g., NEB, acidosis, etc.) that are known to impact animal health, milk production, and/or milk quality. For example, process 400 can use milk quality information and/or activity data to determine whether a cow is in a negative energy balance (NEB) state in which the amount of energy being used for basal metabolic activity and milk production exceeds the energy available from feed ingested by the cow. In a more particular example, process 400 can determine a likely metabolic state of each animal based on milk quality information and, in some examples, other data (e.g., data received at 406) using techniques described below in connection with process 600 of FIG. 6.

As another example, process 400 can use milk quality information and other data to determine whether a cow is experiencing acidosis (e.g., subacute ruminal acidosis (SARA)). SARA can significantly impact milk composition due to alterations in rumen microbial fermentation caused by a drop in rumen pH when fed high-concentrate diets, which can lead to a reduction in milk fat content of milk produced by the animal. This can lead to reduced milk quality (e.g., a decrease in milk fat percentage) and potentially impact overall milk production as well. SARA may also lead to changes in milk protein levels and increased incidence of milk somatic cell counts. In such an example, process 400 can use milk quality information and/or activity data to determine whether a cow is likely to be experiencing SARA or clinical acidosis based on fat content of the milk (e.g., compared to average milk fat for the animal, average milk fat for the herd, etc.). Additionally, process 400 can use other data to determine whether a cow is likely to be experiencing acidosis, such as milk protein levels, milk somatic cell counts, feeding data, etc.

As yet another example, process 400 can use milk quality information and other data to determine whether the feed that a cow has been consuming is resulting in sub-optimal milk production and/or milk quality. In such an example, if a dairy operator wishes to produce milk with a high fat content, a high protein content, high volume, etc., process 400 can use the milk quality information (e.g., including fat content and/or protein content of the milk) and other data to determine whether the amount and/or composition of the feed being consumed by a particular cow is resulting in the milk being produced having the desired milk quality or qualities, and adjusting the feed (e.g., the amount and/or composition) provided to a particular cow (or group of cows) to increase a likelihood that the objective is achieved. The potential of a dairy animal is based on both heredity (~55%) and environmental factors (~45%), such as feeding management, and whether a change in environmental factors, such as feed provided to the animal, can be based on whether the animal is producing milk at, below, or above its estimated potential. In such an example, if a particular cow is producing sub-optimal milk (e.g., relative to a desired quality or qualities of the milk), process 400 can be used to identify whether an adjustment to the care of the cow (e.g., an adjustment to the composition of the feed being consumed by the cow to increase an amount of feed that encourages production of higher fat milk) can be made that is likely to lead to an improvement in the quality of milk produced by the cow. In a more particular example, if milk fat content is sub-optimal, feed composition and quantity can be adjusted (e.g., via an increase in forage quality and quantity, adding fat supplements such as protected fats, etc.). Additionally, process 400 can evaluate the ruminant health of the animal and/or indicate that the ruminant health of the animal should be evaluated (e.g., by a user, veterinarian, etc.). In another more particular example, if milk protein content is sub-optimal, feed composition and quantity can be adjusted (e.g., via an increase in dietary protein level by adding higher protein concentrates, which can ensure that the animal's diet includes adequate rumen degradable protein (RDP)).

Alternatively, if a particular cow is producing milk with at least a desired level of quality (e.g., relative to a desired quality or qualities of the milk, such as desirable milk fat composition characteristics), process 400 can be used to identify whether an adjustment to the care of the cow (e.g., an adjustment to the composition of the feed being consumed by the cow to decrease an amount of feed that encourages production of higher fat milk) can be made that is likely to lead to a reduction in costs associated with feeding of the cow without significantly comprising the quality of the milk produced by the cow. In a more particular example, if milk fat content is high relative to a desired fat content (e.g., a total fat content, fat content relative to protein, etc.), feed composition and quantity can be adjusted (e.g., via an increase in the proportion of concentrate in the animal's diet). Additionally, process 400 can monitor the level of readily fermentable carbohydrates in the feed of the animal, and/or indicate that a user should monitor such levels. In another more particular example, if milk protein content is high relative to a desired protein content, feed composition and quantity can be adjusted (e.g., via an increase in the amount of fiber sources in the forage, via an increase in the level of bypass protein, sometimes referred to as undegradable protein, in the diet). In yet another more particular example, if the ratio of milk protein to milk content is at an undesirable level (e.g., 0.80 in Holsteins), feed composition and quantity can be adjusted to improve the protein to fat ratio (e.g., via an adjustment to decrease the fat content, an adjustment to increase the protein content, or both).

At 410, process 400 can identify any of the animals that have been determined (e.g., at 408) to be experiencing an adverse metabolic condition that is treatable without user intervention. For example, process 400 can determine, for each animal that has been determined to be experiencing an adverse metabolic condition (e.g., at 408), a likelihood that the condition can be resolved by adjustments that can be carried out autonomously, such as changing an amount and/or composition of feed provided to the animal. In a more particular example, if a particular cow is in (or approaching) an NEB state, process 400 can determine whether the energy deficit can be treated without user intervention (e.g., without exceeding a threshold). In some embodiments, if process 400 determines that the metabolic condition of the animal cannot be resolved autonomously and/or if the metabolic condition of animal is associated with a significant likelihood of adverse effects that require human intervention (e.g., by an operator of the dairy, by a veterinarian, etc.), process 400 can attempt to alert a user to the condition of the animal (e.g., by causing an alert to be presented to a user).

As described above, incidence of a metabolic condition, such as NEB, in a herd can be estimated based on testing of a statistically significant sample of animals (e.g., at least twelve animals at once). In some embodiments, process 400 can use the milk quality information from animals milked during a particular period of time (e.g., one morning, one day, etc.) to estimate the incidence of NEB, and/or one or more other metabolic conditions, in the herd based on the milk quality collected information, and can use the incidence in the herd to determine a likelihood that one or more animals are experiencing the metabolic condition have not been detected. For example, process 400 can determine the incidence of NEB in the herd based on a sample of cows milked within the past day, and determine the number of cows that have been recently determined to be to be currently experiencing NEB (e.g., based on a number of cows with a milk fat-to-protein (F/P) ratio below a particular threshold within the past few days, such as the past three, four, or five days). In such an example, process 400 can determine whether there are likely to be a significant number of cows experiencing the metabolic condition (e.g., subclinical NEB) that have gone undetected based on the number of NEB cows that have been identified from recent milk quality information and the incidence in the herd based on the statistically significant sample. If the number of NEB cows that have been identified from recent milk quality information is not similar to the expected number (e.g., within a predetermined range), process 400 can attempt to alert a user to the number of animals that may have an undetected metabolic condition (e.g., by causing an alert to be presented to a user). Additionally or alternatively, process 400 can identify one or more animals that may be experiencing an undetected metabolic condition that can be detected via monitoring of milk from the animal. For example, process 400 can identify (e.g., to a user, to a livestock management system) any cows for which recent milk quality information has not been collected (e.g., cows that have not been milked at an inline-sensor-equipped milking station recently), and a user (and/or automated farm equipment) can cause a sample to be collected from the identified cows (e.g., by directing the cows to milking stations equipped with inline sensors, by manually collecting a sample from the cows, etc.). As another example, process 400 can identify any cows for which recent milk quality information has not been collected and that have other indications of the metabolic condition (e.g., based on activity information), and a user (and/or automated farm equipment) can cause a sample to be collected from the identified cows.

At 412, process 400 can adjust (e.g., without user intervention, or in response to user intervention,) one or more variables associated with care of a particular animal(s) that is experience an adverse metabolic condition based on the metabolic condition milk quality information. In some embodiments, the adjustment can be an adjustment that is expected to alleviate and/or prevent the adverse impact of the identified metabolic condition on animal health, milk production, and/or milk quality.

In some embodiments, process 400 can use any suitable technique or combination of techniques to determine how to adjust a variable(s) based on the milk quality information. For example, in some embodiments, process 400 can determine an adjustment to the amount and/or composition of feed that is being provided to an animal that is expected to address the metabolic condition. In a more particular example, if process 400 determines that a particular cow is experiencing, and/or is likely to, an NEB state, process 400 can determine at 400 an amount(s) (e.g., a dose) of a nutritional supplement (e.g., propylene glycol) to add to the cow's feed each day and a number of days (or feedings) for which the supplement is to be added. In such an example, the amount that is added can be based on an estimation of the degree of energy imbalance and a quantity of supplement that is likely to adequately address the imbalance. Alternatively, the amount to be added can be fixed (e.g., at a level set by a user, such as a dairy operator or veterinarian). Additionally, in some embodiments, the dose can be tapered off over time. In such embodiments, the length and/or amount of tapering can be based on data collected for the cow subsequent to administering the treatment, and a determination of whether the metabolic condition has been addressed. Alternatively, in such embodiments, the length and/or amount of tapering can be predetermined (e.g., based on input from a user, such as a dairy operator or veterinarian, based on the initial dose, etc.). In some embodiments, at 412, process 400 can instruct one or more automated farm equipment systems (e.g., an automated feed system) to implement the adjustment.

As another example, process 400 can determine whether to adjust an amount and/or composition of feed being provided to a particular cow based on the yield, the fat content, and/or protein content of the milk from a milking or multiple milkings. As a more particular example, if the milk quality data indicates that a cow's milk is low in fat or protein (e.g., compared to average fat and/or protein content across a group of cows that includes the compared to an desired or expected fat or protein content for that cow, compared to the historic average for the cow, etc.), the amount of feed that the cow receives can be adjusted to ensure that the cow is getting sufficient nutrients to boost milk production and/or improve milk composition. As another more particular example, if milk urea nitrogen (MUN) levels are high, it can indicate that the cow is not efficiently utilizing protein, and the composition of the feed can be adjusted (e.g., by adding more fiber, or energy-dense foods, by decreasing protein) to help the cow more efficiently utilize the nutrients, which can be expected to produce higher-quality milk.

As yet another example, process 400 can determine whether to adjust how often a cow is milked. In a more particular example, if milk quality data includes a dip in milk yield, the milking frequency can be adjusted to increase how often the cow is milked (e.g., increasing the number of milkings per day, decreasing the interval between milkings, etc.), which can help increase milk production and/or relieve pressure on the udder.

As still another example, process 400 can determine whether to further evaluate the health of the cow and/or treat the cow for a health condition determined from the milk quality data. In a more particular example, when milk quality starts to decline, it can be a signal that the health of the cow is in decline (e.g., if other sources of decline are not evident from the data), such as if the milk has elevated MUN, and/or if the milk temperature is elevated (e.g., relative to a baseline(s), such as a temperature of milk from other cows milked during the same time period), it can indicate that the cow is experiencing mastitis and/or other health issues, and a user can be alerted to the potential declining health of the cow (e.g., the information can be provided to an operator at the dairy and/or a veterinarian, and the user and/or veterinarian can more closely evaluate and/or monitor the health of the cow, begin treating an identified condition, change the routine of the cow, etc., to improve the health of the cow). In another more particular example, if the composition of the milk produced by the cow indicates that the cow is experiencing a nutritional deficiency (e.g., a negative energy balance), a supplement (e.g., a high energy component of fed, such as propylene glycol) can be added to the cow's feed to address the nutritional deficiency.

As a further example, process 400 can determine whether changes in environmental conditions may be causing milk quality issues, such as if external factors, such as temperature, humidity, or stress are likely to impact the milk quality and/or yield of a cow (e.g., if cows are uncomfortable, it can affect how the cows produce milk). In such an example, if milk quality has declined (and/or if measured environmental conditions have changed), it can indicate that environmental conditions are sub-optimal (e.g., in addition to one or more other potential causes of decline in milk quality, and/or as a primary cause of decline in milk quality (e.g., if other sources of decline are not evident from the data). In such an example, adjustments can be made to improve barn conditions, such as setting up or activating a cooling system(s), changing how cows are housed (e.g., adjusting the density of the animal population), etc., to help cows feel more comfortable, which can lead to improvements in milk quality.

As another further example, process 400 can determine whether to suggest or consider a particular breeding decision for a cow based on milk quality over time. In a more particular example, if the quality of milk produced by a cow is generally poor (e.g., there are persistent and/or recurring indications of health and/or metabolic problems over time that are not economically resolved with treatment), a user can deprioritize breeding of that cow (e.g., by focusing breeding on cows that consistently produce higher-quality milk and/or that do not have persistent and/or recurring health and/or metabolic problems, by culling the cow, etc.). As another more particular example, if the quality of milk produced by a cow is generally high (e.g., the cow consistently produces high quality milk, the cow does not have persistent and/or recurring health and/or metabolic problems, the cow quickly recovers from health and/or metabolic problems, etc.), a user can prioritize breeding of that cow.

At 414, process 400 can cause any animals being treated autonomously to be more closely monitored to determine whether the intervention is causing an improvement in the metabolic condition of the animal. For example, process 400 can identify animals that are being treated, and cause the animals to be milked at least a minimum number of times at a milking station equipped with an inline sensor that can be used to monitor milk quality (e.g., via an indication to a user that the animals be milked at a milking station equipped with an inline sensor, by not admitting the animal to milking stations that are not equipped with an inline sensor, etc.). As another example, process 400 can identify animals that are being treated, and can cause feeding and/or consumption of feed by those animals to be more closely monitored to determine whether the animal is receiving the feed that includes the treatment.

In some embodiments, process 400 can omit 414, for example, where the amount of data that is expected to be collected for each animal is likely to be sufficient to evaluate the effectiveness of the intervention. As another example, process 400 can omit 414 where the intervention is expected to be efficacious and/or where increased monitoring is not possible (e.g., if the dairy operation is not equipped to direct particular animals to particular milking stations).

FIG. 5 shows an example of a process 500 for automated milk quality monitoring using inline sensors in accordance with some embodiments of the disclosed subject matter.

At 502, process 500 can receive milk characteristic data from multiple inline sensor devices that each generated the milk characteristic data during a milking session of livestock at a particular time. Additionally or alternatively, in some embodiments, at 504, process 500 can receive raw sensor outputs from multiple inline sensor devices, and calculate milk characteristic data from the raw sensor outputs.

In some embodiments, milk characteristic data and/or raw outputs can be received periodically (e.g., at regular and/or irregular intervals) during a milking session as values are generated. For example, the inline sensors can transmit milk characteristic data and/or raw outputs as the data and/or raw outputs are generated during a milking session. As another example, the inline sensors can transmit milk characteristic data and/or raw outputs at predetermined intervals. Additionally or alternatively, in some embodiments, milk characteristic data and/or raw outputs can be received when a milking session is complete. For example, the inline sensors can transmit milk characteristic data and/or raw outputs for an entire milking session after a milking device has completed milking a particular cow. In some embodiments, process 500 can receive milk characteristic data and/or raw outputs from multiple inline sensor devices concurrently (e.g., data from two inline sensors generated at about the same time can be received at about the same time).

In some embodiments, timing information can be associated with received milk characteristic data and/or raw outputs from an inline sensor. For example, process 500 can associate a time at which milk characteristic data and/or raw outputs were received with the data. As another example, milk characteristic data and/or raw outputs can be associated with a time stamp (e.g., generated by the inline sensor).

In some embodiments, identifying information of an inline sensor that generated milk characteristic data and/or raw outputs can be associated with the received milk characteristic data and/or raw outputs from an inline sensor. For example, different inline sensors can transmit information using different communication channels (e.g., different wired or wireless connections, different ports, different destination addresses, etc.), which can be used to identify a source of data received via a particular communication channel. As another example, different inline sensors can be assigned different address information (e.g., a unique IP address, a unique MAC address, etc.), which can be used to identify a source of data received from a data source.

At 504, process 500 can associate the received milk characteristic data from each sensor device with a particular animal. For example, in some embodiments, process 500 can associate data received from a particular sensor with a particular animal based on which animal was being milked at the milking station associated with the particular sensor at the time when the data was generated by the sensor. In such an example, process 500 can receive information indicating which animal is being milked at a particular milking station, and/or which milking station is being used to milk a particular animal, from any suitable device or system (e.g., devices and/or systems described above in connection with FIG. 1A).

At 506, process 500 can determine at least milk composition data for a particular milking session for a particular animal based on the milk characteristic data for the particular milking session. In some embodiments, process 500 can use milk characteristic data to determine milk composition data using any suitable technique(s). For example, if milk characteristic data is milk composition data of milk flowing through an inline sensor at a particular time, process 500 can use data about the volume of milk flowing through the inline sensor a time when the milk characteristic data was generated to determine milk composition data for a period of time.

In some embodiments, process 500 can determine any suitable milk composition data at 506, such as one or more of fat content, protein content, lactose content, somatic cell concentration, etc. Additionally, in some embodiments, process 400 can determine any other suitable values indicative of milk quality based on the received milk characteristic data, such as whether and/or in what concentration one or more contaminants, metabolites, analytes, etc., are present, a volume of milk produced during the session, etc.

At 508, process 500 can determine milk quality information for each animal in a group of animals based on milk composition data from multiple milking sessions during a particular period of time. In some embodiments, the milk quality information can be based on milk composition data from any suitable number of milking sessions and/or over any suitable period of time. In some embodiments, the milk quality information can include one or more milk quality metrics, such as fat content for each milking session, a value indicative of how the fat content for each milking session compares to average fat content across any suitable population (e.g., all cows, other cows in the herd, etc.), a value indicative of how the fat content for each milking session compares to average fat content of milk produced by the particular cow, average fat content for a set of milking sessions, variance in fat content across milking sessions, etc.

At 510, process 500 can determine, for each animal, a confidence metric for the milk quality information corresponding to the particular period of time based at least in part on the amount of milk characteristic data that was collected during the particular period of time. In some embodiments, process 500 can use any suitable technique(s) to determine a confidence metric for milk quality information. In some embodiments, the confidence metric can be expressed using any suitable technique or combination of techniques (e.g., as a percentage confidence, a margin of error, error bars, etc.). In some embodiments, process 600 can omit 510, for example, where the system and/or devices being used to execute process 500 (e.g., inline sensors 102, milk quality analysis system 114) has been certified as complying with a particular standard (e.g., one or more ICAR standards). In such an example, if the amount of data that has been collected for a particular cow is insufficient to determine a milk quality metric for a particular period of time, process 500 can indicate that the milk quality information does not comply with the standard and/or can inhibit the data from being presented for that period of time.

At 512, process 500 can record milk quality information and associated confidence metrics to facilitate evaluation of animal health, milk production, and/or milk quality (e.g., as described above in connection with process 400 of FIG. 4). For example, process 500 can cause milk quality information and associated confidence metrics to be stored in memory of a computing device executing process 500 and/or any other suitable computing device (e.g., a computing device implementing milk quality monitoring system 114 and/or metabolic condition monitoring system 124).

FIG. 6 shows an example of a process 600 for automated detection of adverse metabolic conditions based on milk quality information in accordance with some embodiments of the disclosed subject matter.

At 602, process 600 can receive, for a particular animal, stored milk quality information and other data for a predetermined period of time. For example, a single measurement of milk quality by an inline sensor is generally sufficient to identify NEB in a cow. As another example, if milk quality information for multiple milkings is stored, process 600 can receive any suitable portion of the information that may be useful in determining whether the cow in experiencing NEB. As a more particular example, multiple milk quality measurements indicating NEB in an animal can confirm the diagnosis (e.g., for multiple measurements that occur close together in time), but is generally not necessary to reliably identify a cow experiencing NEB that is likely to benefit from intervention. Note that while milk quality can be measured over an entire lactation (e.g., each time a cow is milked at a milking station equipped with an inline sensor), some metabolic conditions are more likely to occur during certain periods of a lactation, such as the periparturient period. Additionally, a single cow can have more than one non-consecutive episode during a single lactation period.

In some embodiments, other data that can be received at 602 can include activity data, such as data indicative of how active the cow is, data indicative of time spent ruminating, and data indicative of time spent eating can support a diagnosis of NEB in a particular cow.

At 604, process 600 can analyze the received milk quality information and other data to determine whether the animal is in a negative energy balance (NEB) condition and/or is likely to enter an NEB condition within a predetermined period of time. For example, the milk quality information can be analyzed to determine the milk fat-to-protein (F/P) ratio of the milk for any suitable period of time (e.g., a most recent milking session, a most recent day, etc.), and can compare the F/P ratio to one or more thresholds to determine whether the cow is likely to be experiencing NEB and/or is at increased risk for ketosis.

In a more particular example, a F/P ratio between 1.0 and 1.5 is considered normal for Holstein cows, and a ratio greater than 1.5 increases the risk of ketosis. Note that the normal range does vary in different breeds, and may also vary based on environmental factors (e.g., diet), though the relationship between diet and risk of ketosis is not currently well established. In such an example, if the F/P ratio is greater than 1.5 (or another suitable threshold level, e.g., if the cow is a different breed), process 600 can determine that the animal is likely experiencing an NEB condition.

As another more particular example, Holstein cows that produce milk with an F/P ratio above 1.38 are 2.1 times more likely to become clinically ketotic during the lactation period. In such an example, if the F/P ratio is greater than 1.38 (or another suitable threshold level, e.g., if the cow is a different breed), process 600 can determine that the animal is likely experiencing an NEB condition.

As yet another more particular example, if the F/P ratio is below about 1.38, process 600 can determine that the animal is unlikely to be experiencing an NEB condition; if the F/P ratio is between about 1.38 and about 1.50, process 600 can determine that the animal is likely to be experiencing an NEB condition at first level (e.g., a mild NEB), and if the F/P ratio is above about 1.50, process 600 can determine that the animal is likely to be experiencing an NEB condition at second level (e.g., an elevated NEB, which may still be subclinical).

As still another more example, if the F/P ratio is below about 1.38, process 600 can determine that the animal is unlikely to be experiencing an NEB condition; if the F/P ratio is between about 1.38 and about 1.50, process 600 can use additional data to determine a likelihood that the animal is experiencing an NEB condition (e.g., based on activity, diet, etc.), and if the F/P ratio is above about 1.50, process 600 can determine that the animal is likely to be experiencing an NEB condition (e.g., regardless of other data, such as activity, diet, etc.).

If process 600 determines that the animal is not in an NEB condition and is unlikely to enter an NEB condition within a predetermined period of time ("NO" at 606), process 600 can move to 614, and can end.

Otherwise, if process 600 determines that the animal is in an NEB condition or is likely to enter an NEB condition within a predetermined period of time ("YES" at 606), process 600 can move to 618.

At 608, process 600 can determine a likely impact on the health, milk production, and/or milk quality of the animal if the NEB condition is untreated. For example, based on a comparison of an F/P ratio to a threshold(s), and potentially other activity, process 608 can determine whether, and/or by how much, milk production and/or milk quality of the animal are likely to decline over any suitable time horizon. In a more example, if a Holstein cow is producing milk with an F/P ratio above 1.5, process 600 can determine that the cow is likely to experience a decline in milk production and/or milk quality within a relatively short time (e.g., within days). As another more example, if a Holstein cow is producing milk with an F/P ratio between 1.38 and 1.5, process 600 can determine that the cow has an increased likelihood of experiencing a decline in milk production and/or milk quality within an intermediate time (e.g., within days or weeks).

As another example, process 600 can use a model (e.g., a regression model, a machine learning model, etc.) that uses data indicative of F/P ratio and other data as input to determine whether the impact on the animal is likely to necessitate intervention. In such an example, an output of the model can include any suitable indication(s) of a likely impact on animal health, milk production, and/or milk quality, such as a prediction of milk production and/or milk quality in the future (e.g., the near future), or an estimated likelihood that the animal develops clinical ketosis.

If process 600 determines that a likely impact of the NEB condition does not exceed a threshold impact ("NO" at 610), process 600 can move to 614, and can end. For example, the metabolic condition of the animal can continue to be monitored to detect a change (e.g., an unanticipated change, a change from a level at which a likelihood of progression is uncertain) in the metabolic condition that may begin exceeding an acceptable impact. For example, if the animal is currently experiencing relatively mild NEB condition and/or is unlikely to progress to a clinical NEB condition (e.g., based on the F/P ratio being between 1.38 and 1.5 in a Holstein, based on an estimated likelihood of progression being below a threshold, such as 50%, etc.), process 600 can determine the impact of the detected NEB does not call for intervention.

As another example, if the animal was previously experiencing relatively mild NEB condition and/or is was unlikely to progress to a clinical NEB condition, and milk production, quality, and/or other data indicate that the NEB condition is abating and/or has not progressed (e.g., based on an indication that eating time and/or rumination time for the animal has increased since a subclinical NEB was first detected, based on the F/P ratio decreasing since a subclinical NEB was first detected, etc.), process 600 can determine the impact of the detected NEB does not call for intervention.

As yet another example, if the animal was previously experiencing relatively mild NEB condition and/or is was unlikely to progress to a clinical NEB condition, and milk production, quality, and/or other data indicate that the NEB condition is progressing (e.g., based on an indication that eating time and/or rumination time for the animal has not increased since a subclinical NEB was first detected, based on the F/P ratio increasing since a subclinical NEB was first detected, etc.), process 600 can determine the impact of the detected NEB calls for intervention.

In some embodiments, whether intervention is called under a particular set of conditions can be based on user preferences and/or an analysis of the expected cost of intervention and the expected benefit of intervention. For example, even if a cow is likely to recover from a particular NEB without intervention, intervention (e.g., as described below in connection with 612) is likely to cause the cow's milk production to increase in the short term. In such an example, the cost of the intervention (e.g., the monetary cost associated with adding a supplement to feed for the cow, costs associated with more closely monitoring the animal, etc.) can be compared to the benefit that is likely to be realized by the intervention (e.g., a benefit of avoided costs that may otherwise be incurred if the NEB progresses to clinical NEB, a benefit associated with increased milk production), and determine whether to call for intervention based on whether the predicted benefit of intervention outweighs the cost of intervention.

Otherwise, if process 600 determines that a likely impact of the NEB condition exceeds a threshold impact ("YES" at 610), process, process 600 can move to 612.

At 612, process 600 can determine an intervention that is expected to alleviate and/or prevent an adverse impact of the NEB condition on the health, milk production, and/or milk quality of animal. For example, the length (e.g., a number of doses) of an intervention that includes providing a dose of 300 ml of propylene glycol for three to five days can be determined based on current milk quality, milk production, and/or animal health data, and/or a trend(s) in milk quality, milk production, and/or animal health data. In such an example, the number of days for which treatment is provided can be based on the severity of the current NEB, and/or the likelihood that the health animal and/or milk quality of the animal will be monitored during the course of the treatment (e.g., a likelihood that the animal will be milked at a milking station equipped with an inline sensor, etc.). In a more particular example, if the F/P ratio is above a threshold (e.g., above 1.5 in a Holstein) and additional monitoring is relatively unlikely over the next three days (e.g., based on the number of milk stations equipped with inline sensors), process 600 can determine that a 300 ml dose of propylene glycol is to be provided for five days, unless additional data indicates that the intervention can be stopped before the full five days (e.g., if the cow was milked on the third or fourth day of treatment and has improved a significant amount). As another more particular example, if the F/P ratio is above a first threshold and below a second threshold (e.g., between about 1.38 and 1.5 in a Holstein), process 600 can determine that a 300 ml dose of propylene glycol is to be provided for three days, unless additional data indicates that the intervention should be extended to a full five days (e.g., if the cow was milked on the third day of treatment and has not improved by a significant amount and/or has progressed toward clinical NEB). As yet another example, if a cow has been receiving treatment (e.g., one or more doses of propylene glycol were provided to, and consumed by, the animal on one or more prior days), process 600 can determine whether to provide additional treatment (e.g., to extend the number of doses to be provided) or stop treatment (e.g., to inhibit one or more scheduled future doses from being provided) based on whether, and/or an extent to which, the health, milk quality, and/or milk production of the animal has improved (or declined).

As another example, the type of intervention that is to be provided to the animal can be based on current milk quality, milk production, and/or animal health data, and/or a trend(s) in milk quality, milk production, and/or animal health data. Whether to alert a user and/or a veterinarian to the condition of the animal, and/or prompt a user and/or a veterinarian to evaluate the condition of the animal, can be based on the severity of the current NEB, and/or the likelihood that the health animal and/or milk quality of the animal will be monitored during the course of a treatment (e.g., a likelihood that the animal will be milked at a milking station equipped with an inline sensor, etc.). In a more particular example, if the F/P ratio is significantly elevated (e.g., above 1.5 in a Holstein) and additional automated monitoring is relatively unlikely to occur within a relatively short period of time (e.g., within the next three days), process 600 can determine that a user and a veterinarian is to be alerted to the condition of the animal and/or is to be prompted to more closely monitor and/or evaluate the health of the animal. As another more particular example, if the F/P ratio is above a first threshold and below a second threshold (e.g., between about 1.38 and 1.5 in a Holstein), process 600 can determine that automated intervention is sufficient (e.g., a user may be able to access information indicating that the animal is being treated and/or has recently been treated, but may not be specifically alerted or prompted to act). As yet another example, if a cow has been receiving treatment (e.g., one or more doses of propylene glycol were provided to, and consumed by, the animal on one or more prior days), and the animal's condition is progressing or not significantly improving, process 600 can determine that a user and a veterinarian is to be alerted to the condition of the animal and/or is to be prompted to more closely monitor and/or evaluate the health of the animal.

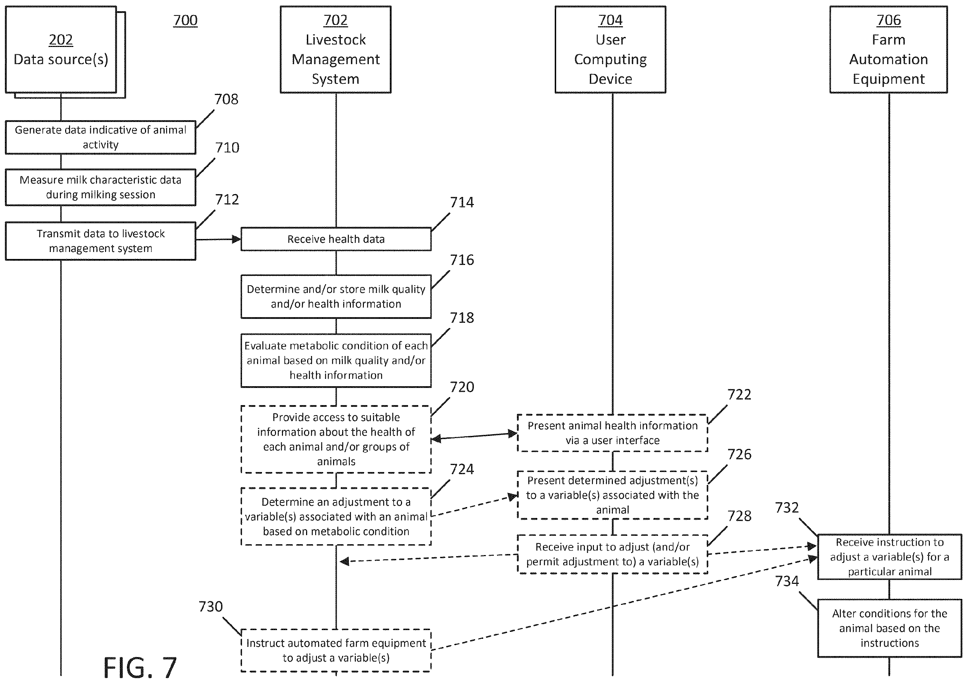

FIG. 7 shows an example flow 700 for automated metabolic condition monitoring and treatment in accordance with some embodiments of the disclosed subject matter. In FIG. 7, example flow 700 can include various devices, such as data sources 202 (e.g., one or more of inline sensors 102 described above in connection with FIGS. 1A and 1B, installed on a subset of milk lines, one or more activity monitoring sensors 122 described above in connection with FIG. 1A), a livestock management system 702 (e.g., including one or more of processors 324 and 344), a user computing device 704 (e.g., a smartphone, a tablet computer, a laptop computer, a desktop computer, a wearable computing device, etc.), and farm automation equipment 706 (e.g., as described above in connection with FIGS. 1A, 1B, and 4).

At 708, data sources 202 can generate data indicative of animal activity (e.g., data that can be used to determine a position, posture, and/or activity of an animal, such as a dairy cow, as described above in connection with FIGS. 1A and 4) that can be used to determine one or more metabolic condition metrics (e.g., as described above in connection with FIGS. 1A to 4).

At 710, data sources 202 can generate milk quality data (e.g., raw measurement data, milk characteristic information determined based on raw measurement data) during a milking session that can be used to determine milk quality metrics.

At 712, data sources 2202 can transmit health data (e.g., raw and/or processed activity data, metabolic condition metrics, raw measurement data, milk characteristic information determined based on raw measurement data) to livestock management system 702 (e.g., via a local wireless connection, via a dedicated wired connection, via a LAN, via a WAN, etc.). As described above, a livestock management system can be implemented locally and/or remotely, and accordingly, transmission of data by data sources 202 at 712 can include transmission to a local device (e.g., a local computing device, such as local computing device 220) or a remote device (e.g., a dedicated remote server, a cloud computing service, etc.).

At 714, livestock management system 702 can receive health data generated by data sources 202, and at 716, livestock management system 702 can determine and/or store milk quality information and/or any other suitable health information (e.g., metabolic condition information) in connection with a particular animal associated with the data (e.g., based on a determination that the particular animal was milked using a milking station associated with the sensor while the data was being generated, based on a device(s) used to generate the data such as identifying information of a transmitter affixed to the animal that was used to determine activity of the animal).

At 718, livestock management system 702 can evaluate a metabolic condition of each animal based on the milk quality information and/or other health information associated with the animal. For example, livestock management system 702 can use techniques described above in connection with 408 of FIG. 4 and/or process 600 of FIG. 6 to determine evaluate a metabolic condition of the animal.

At 720, livestock management system 702 can provide access to any suitable information about the health of each animal being monitored and/or for one or more groups of animals (e.g., a subset of cows in a herd that share a particular characteristic). For example, livestock management system 702 can store milk quality information and metabolic condition information, and an authorized user can be permitted to access stored health information using a user computing device (e.g., via a webpage that facilitates access to data stored by livestock management system 702, via an installed application such as a mobile application that that facilitates access to data stored by livestock management system 702, via an authorized application program interface (API) call from a computing device, etc.).

At 722, user computing device 704 can present animal health information accessed from livestock management system 702 via a user interface (e.g., a graphical user interface). In some embodiments, flow 700 can omit 720 and 722, for example where a user permits livestock management system 702 to initiate adjustments to one or more variables associated with care of a particular animal autonomously (note, however, that users generally can be permitted to access the health information, regardless of whether adjustments are carried out autonomously).

At 724, livestock management system 702 can determine an adjustment to a variable associated with a particular animal based on milk quality information and/or metabolic condition information (e.g., as described above in connection with 412 of FIG. 4 and 612 of FIG. 6). For example, livestock management system 702 can determine an adjustment that is likely to: improve the health of the animal; prevent the health of the animal from deteriorating; improve milk production by the animal; etc.). In some embodiments, livestock management system 702 can prompt a user to review and/or authorize an adjustment that is to be made. In such embodiments, livestock management system 702 can cause a user computing device (e.g., user computing device 704) to present information about the adjustment determined at 724 (e.g., by transmitting an alert via a push notification to a user computing device(s) associated with a particular user(s), a text message, an email, etc.). In some embodiments, flow 700 can omit 724, for example where a user does not permit livestock management system 702 to suggest adjustments to one or more variables associated with care of animals autonomously.

At 726, user computing device 504 can present information about an adjustment determined at 724 (e.g., via a graphical user interface). In some embodiments, the information about the adjustment can include any suitable information, such as identifying information of the animal for which the adjustment is to be made, information indicating what prompted the adjustment, at least a portion of the milk quality information for the animal(s) and/or a summary of milk quality information for the animal(s), at least a portion of the metabolic condition information for the animal(s) and/or a summary of metabolic condition information for the animal(s), information describing the proposed adjustment, etc.

At 728, user computing device 704 can receive input to adjust one or more variables associated with a particular animal(s) (e.g., via a GUI). For example, if livestock management system 702 generated a suggested adjustment at 724 that requires authorization, input can be provided at 728 to cause the adjustment to be implemented. As another example, regardless of whether livestock management system 702 generated a suggested adjustment at 724, a user may be permitted to provide input to cause an adjustment to be implemented for one or more animals (e.g., a user can implement adjustments if livestock management system 702 is not configured to automatically determine adjustments and/or if the user desires to make an adjustment that has not been identified by livestock management system 702). In some embodiments, flow 700 can omit 726 and/or 728, for example where a user permits livestock management system 702 to initiate adjustments to one or more variables associated with care of a particular animal autonomously (note, however, that users generally can be permitted to access the health information, regardless of whether adjustments are carried out autonomously).

At 730, livestock management system 702 can instruct one or more automated farm equipment systems to implement an adjustment to the variable(s) determined at 724 and/or input at 728. At 732, automated farm equipment 706 can receive an instruction(s) to adjust a variable(s) for a particular animal(s) from livestock management system 702 and/or user computing device 704. At 734, automated farm equipment 706 can alter conditions for the animal based on the instruction (e.g., by adjusting the amount and/or composition of feed provided to the animal).

### Further Examples Having a Variety of Features

Implementation examples are described in the following numbered clauses:
1. A method for automated detection of metabolic condition of dairy livestock, the method comprising: receiving, from each of a plurality of inline sensors, milk data indicative of milk composition information of milk flowing through that inline sensor during a milking session, wherein each of the plurality of inline sensors is configured to measure milk composition information of milk flowing through one of a plurality of milk lines, which are each associated with a respective milking station of a plurality of milking stations, and wherein a first subset of milk lines of the plurality of milk lines are equipped with a respective inline sensor of the plurality of inline sensors, such that milk composition information is generated for milk flowing through the first subset of milk lines during milking; associating the received milk data with a particular animal that produced the milk; storing milk composition information for milk from the particular animal during the milking session; determining metabolic condition information for the particular animal using stored milk composition information for at least one milking session; and determining, based on the metabolic condition information, that the particular animal is experiencing a subclinical negative energy balance.
2. The method of clause 1, further comprising: in response to determining that the particular animal is experiencing a subclinical negative energy balance and without user intervention, instructing a robotic feeding system to adjust the composition of feed provided to the particular animal to provide additional energy to the particular animal, wherein the robotic feeding system is configured to dispense feed for consumption by each of a plurality of animals, including the particular animal.
3. The method of clause 2, wherein the one or more hardware processors are further configured to: determining a dose of a high energy supplement to provide to the particular animal based on the metabolic condition information; and instructing the robotic feeding system to add an amount of the high energy supplement to feed provided to the particular animal, wherein the amount is based on the dose.
4. The method of clause 3, wherein the high energy supplement comprises propylene glycol.
5. The method of any one of clauses 3 or 4, wherein the high energy supplement consists essentially of propylene glycol.
6. The method of any one of clauses 1 to 5, further comprising: receiving, from an activity monitoring sensor, activity data indicative of activity of the particular animal, wherein the activity monitoring sensor configured to measure animal activity information; storing activity information for the particular animal; determining the metabolic condition information for the particular animal using the stored milk composition information for the at least one milking session and stored activity information for over a predetermined period of time that includes the at least one milking session.
7. The method of clause 6, further comprising: receiving, from the activity monitoring sensor, activity data indicative of activity of a second particular animal; storing activity information for the second particular animal; determining metabolic condition information for the second particular animal using stored milk composition information for a plurality of milking sessions over a predetermined period of time and stored activity information for the predetermined period of time; determining, based on the metabolic condition information, that the particular animal is not experiencing a subclinical negative energy balance; and instructing a robotic feeding system to provide feed to the second particular animal that does not include an added amount of the high energy supplement.
8. The method of any one of clauses 6 or 7, wherein the activity monitoring sensor is affixed to the particular animal, and is one of a plurality of activity monitoring sensors, each associated with a different animal of a plurality of animals.
9. The method of any one of clauses 1 to 8, wherein a second subset of milk lines the plurality of milk lines are not equipped with inline an inline sensor configured to measure milk composition information of milk flowing through one of the second subset of milk lines, such that milk composition information is not generated for milk flowing through the second subset of milk lines during milking, and wherein the number of milk lines in the first subset of milk lines is less than the number of milk lines in the second subset of milk lines.
10. The method of clause 9, wherein each milk line of the plurality of milk lines is a member of the first subset of milk lines of the second subset of milk lines.
11. The method of any one of clauses 1 to 10, wherein the plurality of milking stations comprise all of the milk stations of a milking operation, such that only a fraction of milking stations of the milking operation are equipped with inline sensors configured to measure milk composition information of milk during milking.
12. The method of clause 11, wherein the first subset of milk lines is about thirty percent of the plurality of milking stations.
13. The method of any one of clauses 11 or 12, wherein the milking operation comprises a robotic milking system configured to autonomously attach milking devices to animals at the plurality of milking stations for milking.
14. The method of any one of clauses 1 to 13, wherein the stored milk composition information comprises fat content information for the milking session, protein content information for the milking session, and/or lactose content information for the milking session.
15. A system comprising: at least one processor that is configured to: perform a method of any of clauses 1 to 14.
16. A non-transitory computer-readable medium storing computer-executable code, comprising code for causing a computer to cause a processor to: perform a method of any of clauses 1 to 14.

In some embodiments, any suitable computer readable media can be used for storing instructions for performing functions and/or processes described herein. For example, in some embodiments, computer readable media can be transitory or non-transitory. For example, non-transitory computer readable media can include media such as magnetic media (such as hard disks, floppy disks, etc.), optical media (such as compact discs, digital video discs, Blu-ray discs, etc.), semiconductor media (such as RAM, Flash memory, electrically programmable read only memory (EPROM), electrically erasable programmable read only memory (EEPROM), etc.), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

It should be noted that, as used herein, the term mechanism can encompass hardware, software, firmware, or any suitable combination thereof.

It should be understood that above-described steps of the processes of FIGS. 4 to 6 can be executed or performed in any suitable order or sequence not limited to the order and sequence shown and described in the figures. Also, some of the above steps of the processes of FIGS. 4 to 6 can be executed or performed substantially simultaneously where appropriate or in parallel to reduce latency and processing times.

This written description uses examples to disclose the invention(s), including the best mode, and also to enable any person skilled in the art to make and use the invention(s). Certain terms have been used for brevity, clarity, and understanding. No unnecessary limitations are to be inferred therefrom beyond the requirement of the prior art because such terms are used for descriptive purposes only and are intended to be broadly construed. The patentable scope of the invention(s) is defined by the claims and may include other examples that occur to those skilled in the art. Such other examples are intended to be within the scope of the claims if they have features or structural elements that do not differ from the literal language of the claims, or if they include equivalent features or structural elements with insubstantial differences from the literal languages of the claims.

## Claims

1. A system (100) for automated detection of metabolic condition of dairy livestock, the system (100) comprising:
a plurality of milking stations (106), each associated with a respective milk line of a plurality of milk lines (104);
a plurality of inline sensors (102), each configured to measure milk composition information of milk flowing through one of the plurality of milk lines (104),
wherein a first subset of milk lines of the plurality of milk lines (104) are equipped with a respective inline sensor of the plurality of inline sensors (102), such that milk composition information is generated for milk flowing through the first subset of milk lines during milking; and
one or more hardware processors configured to:
receive, from each of the plurality of inline sensors (102), milk data indicative of milk composition information of milk flowing through that inline sensor during a milking session;
associate the received milk data with a particular animal that produced the milk;
store milk composition information for milk from the particular animal during the milking session;
determine metabolic condition information for the particular animal using stored milk composition information for at least one milking session; and
determine, based on the metabolic condition information, that the particular animal is experiencing a subclinical negative energy balance.

2. The system (100) of claim 1, further comprising:
a robotic feeding system (118) configured to dispense feed for consumption by each of a plurality of animals, including the particular animal,
wherein the one or more hardware processors are further configured to:
in response to determining that the particular animal is experiencing a subclinical negative energy balance and without user intervention, instruct the robotic feeding system (118) to adjust the composition of feed provided to the particular animal to provide additional energy to the particular animal.

3. The system (100) of claim 2, wherein the one or more hardware processors are further configured to:
determine a dose of a high energy supplement to provide to the particular animal based on the metabolic condition information; and
instruct the robotic feeding system (118) to add an amount of the high energy supplement to feed provided to the particular animal,
wherein the amount is based on the dose.

4. The system (100) of claim 3, wherein the high energy supplement comprises propylene glycol, optionally wherein the high energy supplement consists essentially of propylene glycol.

5. The system (100) of any one of the preceding claims, further comprising:
an activity monitoring sensor (122) configured to measure animal activity information,
wherein the one or more hardware processors are further configured to:
receive, from the activity monitoring sensor (122), activity data indicative of activity of the particular animal;
store activity information for the particular animal; and
determine the metabolic condition information for the particular animal using the stored milk composition information for the at least one milking session and stored activity information for over a predetermined period of time that includes the at least one milking session.

6. The system (100) of claim 5, wherein the one or more hardware processors are further configured to:
receive, from the activity monitoring sensor (122), activity data indicative of activity of a second particular animal;
store activity information for the second particular animal;
determine metabolic condition information for the second particular animal using stored milk composition information for a plurality of milking sessions over a predetermined period of time and stored activity information for the predetermined period of time;
determine, based on the metabolic condition information, that the particular animal is not experiencing a subclinical negative energy balance; and
instruct a robotic feeding system (118) to provide feed to the second particular animal that does not include an added amount of a high energy supplement.

7. The system (100) of claim 5 or 6, wherein the activity monitoring sensor (122) is affixed to the particular animal, and is one of a plurality of activity monitoring sensors, each associated with a different animal of a plurality of animals.

8. The system (100) of any one of the preceding claims, wherein a second subset of milk lines of the plurality of milk lines (104) are not equipped with inline an inline sensor configured to measure milk composition information of milk flowing through one of the second subset of milk lines, such that milk composition information is not generated for milk flowing through the second subset of milk lines during milking, and
wherein the number of milk lines in the first subset of milk lines is less than the number of milk lines in the second subset of milk lines, optionally wherein each milk line of the plurality of milk lines (104) is a member of the first subset of milk lines or the second subset of milk lines.

9. The system (100) of any one of the preceding claims, wherein the plurality of milking stations (106) comprise all of the milk stations of a milking operation (120), such that only a fraction of milking stations of the milking operation (120) are equipped with inline sensors (102) configured to measure milk composition information of milk during milking, optionally wherein the first subset of milk lines is about thirty percent of the plurality of milking stations (106).

10. The system (100) of any one of the preceding claims, wherein the milking operation (120) comprises a robotic milking system (118) configured to autonomously attach milking devices to animals at the plurality of milking stations (106) for milking.

11. The system (100) of any one of the preceding claims, wherein the stored milk composition information comprises fat content information for the milking session, protein content information for the milking session, and/or lactose content information for the milking session.

12. A method for automated detection of metabolic condition of dairy livestock, the method comprising:
receiving, from each of a plurality of inline sensors (102), milk data indicative of milk composition information of milk flowing through that inline sensor during a milking session,
wherein each of the plurality of inline sensors (102) is configured to measure milk composition information of milk flowing through one of a plurality of milk lines (104), which are each associated with a respective milking station of a plurality of milking stations (106), and
wherein a first subset of milk lines of the plurality of milk lines (104) are equipped with a respective inline sensor of the plurality of inline sensors (102), such that milk composition information is generated for milk flowing through the first subset of milk lines during milking;
associating the received milk data with a particular animal that produced the milk;
storing milk composition information for milk from the particular animal during the milking session;
determining metabolic condition information for the particular animal using stored milk composition information for at least one milking session; and
determining, based on the metabolic condition information, that the particular animal is experiencing a subclinical negative energy balance.

13. The method of claim 12, further comprising:
in response to determining that the particular animal is experiencing a subclinical negative energy balance and without user intervention, instructing a robotic feeding system (118) to adjust the composition of feed provided to the particular animal to provide additional energy to the particular animal,
wherein the robotic feeding system (118) is configured to dispense feed for consumption by each of a plurality of animals, including the particular animal.

14. The method of claim 13, further comprising:
determining a dose of a high energy supplement to provide to the particular animal based on the metabolic condition information; and
instructing the robotic feeding system (118) to add an amount of the high energy supplement to feed provided to the particular animal,
wherein the amount is based on the dose, optionally wherein the high energy supplement comprises propylene glycol, optionally wherein the high energy supplement consists essentially of propylene glycol.

15. The method of claim 12, 13 or 14, further comprising:
receiving, from an activity monitoring sensor (122), activity data indicative of activity of the particular animal,
wherein the activity monitoring sensor (122) is configured to measure animal activity information;
storing activity information for the particular animal; and
determining the metabolic condition information for the particular animal using the stored milk composition information for the at least one milking session and stored activity information for over a predetermined period of time that includes the at least one milking session.
